(19) Europäisches Patentamt
European Patent Office
Office européen des brevets

(11) EP 0 902 027 B1

(12) **FASCICULE DE BREVET EUROPEEN**

(45) Date de publication et mention de la délivrance du brevet:
**25.07.2001 Bulletin 2001/30**

(51) Int Cl.[7]: **C07D 403/04**, C07D 403/14, A61K 31/41

(21) Numéro de dépôt: **98402154.3**

(22) Date de dépôt: **01.09.1998**

(54) **Nouveaux dérivés de l'indole et de l'indazole, leur procédé de préparation et les compositions pharmaceutiques qui les contiennent**

Indol- und Indazol-derivate, Verfahren zu deren Herstellung und sie enthaltende Zusammensetzungen

Indole and indazole derivatives, process for their preparation and the pharmaceutical compositions containing them

(84) Etats contractants désignés:
**AT BE CH DE DK ES FI FR GB GR IE IT LI LU NL PT SE**

(30) Priorité: **03.09.1997 FR 9710939**

(43) Date de publication de la demande:
**17.03.1999 Bulletin 1999/11**

(73) Titulaire: **ADIR ET COMPAGNIE**
**92415 Courbevoie Cédex (FR)**

(72) Inventeurs:
- **Lavielle, Gilbert**
  **78170 La Celle Saint Cloud (FR)**
- **Muller, Olivier**
  **95300 Ennery (FR)**
- **Vayssettes-Courchay, Christine**
  **91430 Igny (FR)**
- **Descombes, Jean-Jacques**
  **92500 Rueil Malmaison (FR)**
- **Verbeuren, Tony**
  **78540 Vernouillet (FR)**

(56) Documents cités:
**EP-A- 0 135 781**
**EP-A- 0 597 112**
**GB-A- 2 289 465**
**EP-A- 0 454 330**
**WO-A-97/06159**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 0 902 027 B1

## Description

La présente invention concerne de nouveaux dérivés de l'indole et de l'indazole, leur procédé de préparation et les compositions pharmaceutiques qui les contiennent.

De nombreux dérivés de l'indole ont été décrits dans la littérature. Certains d'entre eux ont été étudiés pour leurs propriétés 5-$HT_{1D}$ - 5-$HT_{1\text{-like}}$ agonistes. A titre d'exemple, on peut citer les demandes WO 97/11675, WO 97/06159, WO 96/04269, GB 2289465. D'autres ont été étudiés pour leur pouvoir inhibiteur de synthèse de thromboxane $A_2$ et leurs propriétés antihistaminique, en particulier dans la demande EP 597112. Le brevet EP 135 781 décrit, quant à lui, des dérivés de l'indazole en tant qu'analgésiques centraux ayant des propriétés neuroleptiques.

Les récepteurs de la sérotonine (5-HT) ont été subdivisés en sept familles majeures dont la famille des récepteurs 5-$HT_1$ forme un groupe hétérogène dans laquelle certains récepteurs n'ont pas encore été bien caractérisés.

Le sumatriptan, agent anti-migraineux, interagit avec des récepteurs 5-$HT_1$ qui ont été décrits comme étant les types 5-$HT_{1B}$, 5-$HT_{1D}$ ou 5-$HT_{1\text{-like}}$ (Sumner et al., Brit. J. Pharmacol., 105, 603, 1992, Olesen, La Recherche, 23, 160, 1992). La sélectivité du sumatriptan pour ces récepteurs 5-$HT_{1B,\ 1D,\ 1\text{-like}}$ a été proposée comme la raison principale de son activité anti-migraine (Macon et al., J. Med. Chem., 37, 2509, 1994), dans laquelle la vasoconstriction sélective du lit vasculaire carotidien est impliquée (Saxena et al. T.I.P.S., 10, 200, 1989). De tels récepteurs, présents au niveau du système veineux, se retrouvent également dans le cerveau et leur activation ou inhibition peut être à l'origine de certaines maladies du système nerveux central (Clitherow et al., J. Med. Chem, 37, 2253, 1994).

Les composés de la présente invention possèdent une structure originale due en particulier à la présence d'une chaîne latérale aminée substituant l'azote hétérocyclique et d'un motif triazole substituant le noyau aromatique. Cette structure confère aux dérivés de l'invention, de façon surprenante, une grande sélectivité pour les récepteurs 5-$HT_{1B}$, 5-$HT_{1D}$ ou 5-$HT_{1like}$. Ils sont donc susceptibles d'être utilisés comme agent veinotonique dans le traitement de l'insuffisance veineuse et des maladies associées, mais aussi dans le traitement de la migraine et des conditions associées à cette maladie, des "cluster headaches", de la douleur, de la migraine associée aux maladies vasculaires ainsi que dans l'hypertension, l'obésité et les désordres alimentaires.

L'activité des composés de l'invention a été évaluée dans un test pharmacologique mesurant leur potentiel de contraction de la veine saphène isolée du chien ou du lapin comme décrit par Humphrey et al. (Br. J. Pharmacol., 94, 1123, 1988).

La présente invention concerne les dérivés de formule (I) :

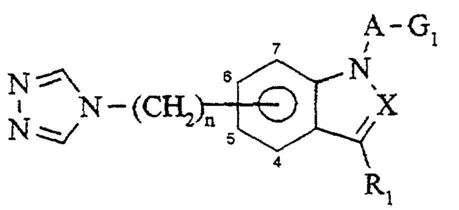

(I)

dans laquelle :

- n est égal à 0 ou 1,
- A représente une liaison σ ou un groupement alkylène ($C_1$-$C_8$) linéaire ou ramifié, ou un groupement alkénylène ($C_2$-$C_8$) linéaire ou ramifié,
- X représente un atome d'azote ou un groupement C-$R_2$ dans lequel $R_2$ représente un atome d'hydrogène, ou un groupement alkyle ($C_1$-$C_6$) linéaire ou ramifié,
- $R_1$ représente un atome d'hydrogène ou un groupement alkyle ($C_1$-$C_6$) linéaire ou ramifié,
- $G_1$ représente un groupement pyrrolidinyle ou pipéridyle, chacun de ces groupements étant éventuellement substitué sur l'un quelconque des sommets du cycle par un groupement alkyle ($C_1$-$C_6$) linéaire ou ramifié, cycloalkyle ($C_3$-$C_7$), cycloalkyle ($C_3$-$C_7$) alkyle ($C_1$-$C_6$) linéaire ou ramifié, nitrile, carboxy, alkoxy ($C_1$-$C_6$) carbonyle linéaire ou ramifié, carbamoyle (éventuellement substitué par un ou deux groupements alkyle ($C_1$-$C_6$) linéaire ou ramifié, cycloalkyle ($C_3$-$C_7$), phényle éventuellement substitué, et/ou benzyle éventuellement substitué), aryle éventuellement substitué, arylalkyle ($C_1$-$C_6$) linéaire ou ramifié éventuellement substitué, hétéroaryle éventuellement substitué, ou hétéroarylalkyle ($C_1$-$C_6$) linéaire ou ramifié éventuellement substitué,

étant entendu que ces groupements pyrrolidine et pipéridine peuvent être liés à A par l'un quelconque des atomes du cycle,
ou bien, $G_1$ représente un groupement

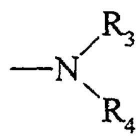

dans lequel :

$R_3$ et $R_4$ représentent indépendamment un atome d'hydrogène, ou un groupement alkyle ($C_1$-$C_6$) linéaire ou ramifié, cycloalkyle ($C_3$-$C_7$), cycloalkyl ($C_3$-$C_7$) alkyle ($C_1$-$C_6$) linéaire ou ramifié, arylalkyle ($C_1$-$C_6$) linéaire ou ramifié éventuellement substitué, aryle éventuellement substitué, ou forment ensemble avec l'atome d'azote qui les porte un groupement :

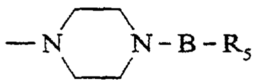 ,

pour lequel B représente une liaison σ ou un groupement alkylène ($C_1$-$C_6$) linéaire ou ramifié, et $R_5$ représente un atome d'hydrogène, ou un groupement alkyle ($C_1$-$C_6$) linéaire ou ramifié, cycloalkyle ($C_3$-$C_7$), aryle éventuellement substitué, arylalkyle ($C_1$-$C_6$) linéaire ou ramifié éventuellement substitué, hétéroaryle éventuellement substitué, ou hétéroarylalkyle ($C_1$-$C_6$) linéaire ou ramifié éventuellement substitué,

leurs énantiomères, diastéréoisomères, ainsi que leurs sels d'addition à un acide ou à une base pharmaceutiquement acceptable.

Par groupement aryle, on entend un groupement choisi parmi phényle et naphtyle.

Par groupement hétéroaryle, on entend un groupement choisi parmi furyle, thiényle, pyridyle, pyrrolyle, imidazolyle, tétrazolyle, pyrazinyle et pyrimidinyle.

Le terme *éventuellement substitué* affectant les expressions phényle, benzyle, aryle, arylalkyle, hétéroaryle, ou hétéroarylalkyle signifie que les groupements concernés sont substitués par un ou plusieurs atomes d'halogène, et/ou groupement alkyle ($C_1$-$C_6$) linéaire ou ramifié, alkoxy ($C_1$-$C_6$) linéaire ou ramifié, hydroxy, et/ou trihalogénoalkyle ($C_1$-$C_6$) linéaire ou ramifié.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

De façon avantageuse, la présente invention concerne les dérivés de formule (I) pour lesquels le groupement triazolyl ou triazolylméthyl est rattaché en position 6 du noyau indole ou indazole.

Un autre aspect avantageux de l'invention concerne les composés de formule (I) pour lesquels le groupement triazolyl ou triazolylméthyl est rattaché en position 5 du noyau indole ou indazole.

Les composés préférés de l'invention sont ceux pour lesquels n vaut 0.

L'invention concerne préférentiellement les composés de formule (I) pour lesquels X représente un groupement $CR_2$, $R_2$ étant préférentiellement un atome d'hydrogène.

D'autres composés préférés, de formule (I) sont ceux pour lesquels X représente un atome d'azote.

Dans les composés de formule (I), $R_1$ représente préférentiellement un atome d'hydrogène.

De façon préférentielle, l'invention concerne les composés de formule (I) pour lesquels dans le substituant A-$G_1$, A représente une liaison σ ou un groupement alkylène ($C_1$-$C_6$) linéaire ou ramifié, et $G_1$ représente un groupement pyrrolidinyle (par exemple 3-pyrrolidinyle), pipéridyle (par exemple 4-pipéridyle) ou 1-pipérazinyle, lesdit groupements étant substitués ou non. Parmi les substituants préférés, on peut citer de façon avantageuse les groupements alkyle ($C_1$-$C_6$) linéaire ou ramifié, aryle, arylalkyle ($C_1$-$C_6$) linéaire ou ramifié, hétéroaryle, et/ou hétéroarylalkyle ($C_1$-$C_6$) linéaire ou ramifié. Les groupements pyrrolidinyle et pipéridyle étant avantageusement substitués sur l'atome d'azote.

Un autre aspect préféré de l'invention concerne les composés de formule (I) pour lesquels A représente un groupement alkylène ($C_1$-$C_6$) linéaire ou ramifié et $G_1$ représente un groupement

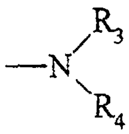

dans lequel $R_3$ et $R_4$ représentent indépendamment un atome d'hydrogène, ou un groupement alkyle ($C_1$-$C_6$) linéaire ou ramifié, cycloalkyle ($C_3$-$C_7$), cycloalkyl ($C_3$-$C_7$) alkyle ($C_1$-$C_6$) linéaire ou ramifié, arylalkyle ($C_1$-$C_6$) linéaire ou ramifié éventuellement substitué, aryle éventuellement substitué. De façon avantageuse $R_3$ et $R_4$ représentent un groupement alkyle ($C_1$-$C_6$) linéaire ou ramifié.

De façon plus préférentielle, l'invention concerne les composés de formule (I) pour lesquels le groupement triazolyle ou triazolylméthyle est rattaché en position 6 du noyau indole ou indazole, X représente un groupement -CH- ou un atome d'azote, $R_1$ représente un atome d'hydrogène, A représente une liaison σ ou un groupement alkylène ($C_1$-$C_6$) linéaire ou ramifié tandis que $G_1$ représente un groupement pyrrolidinyle ou pipéridinyle éventuellement substitués sur l'un quelconque des sommets du cycle par un groupement alkyle ($C_1$-$C_6$) linéaire ou ramifié, aryle éventuellement substitué, arylalkyle ($C_1$-$C_6$) linéaire ou ramifié éventuellement substitué, hétéroaryle éventuellement substitué, ou hétéroarylalkyle ($C_1$-$C_6$) linéaire ou ramifié éventuellement substitué, ou $G_1$ représente un groupement 1-pipérazinyle éventuellement substitué sur l'atome d'azote en position 4 par un groupement alkyle ($C_1$-$C_6$) linéaire ou ramifié, aryle éventuellement substitué, arylalkyle ($C_1$-$C_6$) linéaire ou ramifié éventuellement substitué, hétéroaryle éventuellement substitué, ou hétéroarylalkyle ($C_1$-$C_6$) linéaire ou ramifié éventuellement substitué, ou bien A représente un groupement alkylène ($C_1$-$C_6$) linéaire ou ramifié tandis que G, représente un groupement

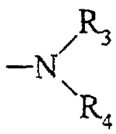

dans lequel $R_3$ et $R_4$ représentent indépendamment un atome d'hydrogène, ou un groupement alkyle ($C_1$-$C_6$) linéaire ou ramifié.

L'invention s'étend également au procédé de préparation des composés de formule (I), caractérisé,

- lorsque dans le composé de formule (I) que l'on souhaite obtenir, X représente un groupement C-$R_2$, en ce que l'on utilise comme produit de départ un composé de formule (II/a):

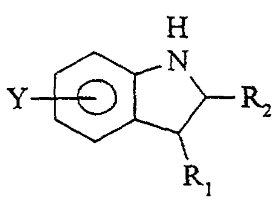

(II/a)

dans laquelle $R_1$ et $R_2$ sont tels que définis dans la formule (I) et Y représente un groupement nitro si dans le composé de formule (I) que l'on souhaite obtenir n vaut 0, ou un groupement hydroxyméthyle si dans le composé de formule (I) que l'on souhaite obtenir, n vaut 1,
qui est condensé,

- ***soit*** sur un dérivé de formule (III/a) :

$$Z - A - G_1 \quad \text{(III/a)}$$

dans laquelle A et $G_1$ sont tels que définis dans la formule (I) et Z représente un atome d'halogène ou un groupement formyle,
pour conduire à un composé de formule (IV) :

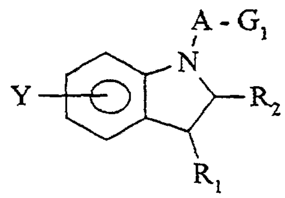

(IV)

dans laquelle A, Y, $G_1$, $R_1$, $R_2$ sont tels que définis précédemment, qui subit une réaction d'oxydation pour conduire à un composé de formule (V/a) :

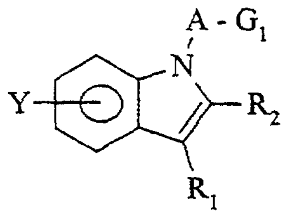

(V/a)

dans laquelle A, Y, $G_1$, $R_1$, $R_2$ sont tels que définis précédemment, dont le groupement $G_1$, lorsqu'il représente un groupement pyrrolidinyle, pipéridyle ou pipérazinyle, peut être substitué sur l'atome d'azote, en milieu basique ou par une réaction d'amination réductrice sur un dérivé carbonylé,

- ***soit*** sur un dérivé de formule (III/b), en milieu basique :

Br - A - OH (III/b)

dans laquelle A a la même signification que précédemment, pour conduire à un composé de formule (IV') :

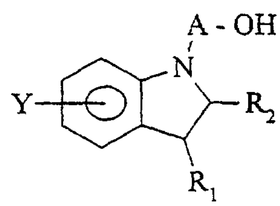

(IV')

dans laquelle Y, A, $R_1$, $R_2$ sont tels que définis précédemment, qui, après bromation et oxydation, est condensé sur un dérivé de formule $G_1H$, $G_1$ ayant la même signification que dans la formule (I), pour conduire à un composé de formule (V/a), telle que définie précédemment,

ou bien,

♦ lorsque dans le composé de formule (I) que l'on souhaite obtenir, X représente un atome d'azote, en ce que l'on utilise comme matière première un composé de formule (II/b):

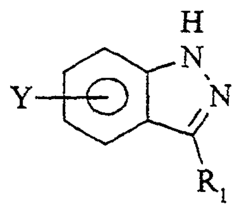

(II/b)

dans laquelle Y et $R_1$ sont tels que définis précédemment,
qui est condensé,

- ***soit*** sur un dérivé de formule (III/c), en milieu basique :

Hal - A - $G_1$ (III/c)

dans laquelle A et $G_1$ sont tels que définis précédemment, et Hal représente un atome d'halogène, pour conduire à un composé de formule (V/b) :

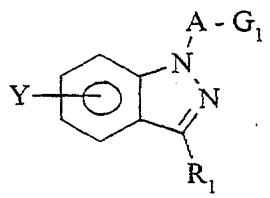

(V/b)

dans laquelle A, Y, $G_1$ et $R_1$ sont tels que définis précédemment,
dont le groupement $G_1$, lorsqu'il représente un groupement pyrrolidinyle, pipéridyle ou pipérazinyle, peut être substitué sur l'atome d'azote, en milieu basique ou par une réaction d'amination réductrice sur un dérivé carbonylé,

- ***soit*** sur un dérivé de formule (III/b) telle que définie précédemment, pour conduire à un composé de formule (VI) :

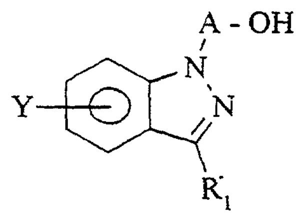

(VI)

dans laquelle Y, A et $R_1$ sont tels que définis précédemment, qui, après bromation, est condensé sur un dérivé de formule $HG_1$, $G_1$ ayant la même signification que dans la formule (I), pour conduire à un composé de formule (V/b) telle que définie précédemment,
composés de formule (V/a) et (V/b) formant la totalité des composés de formule (V) :

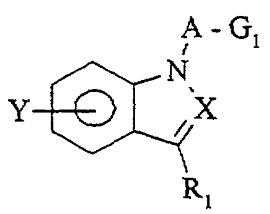

(V)

dans laquelle X, Y, A, $R_1$, et $G_1$ sont tels que définis dans la formule (I), et Y est tel que défini précédemment,
qui, par réduction du groupement Y (lorsque Y représente un groupement hydroxyméthyle après passage par un intermédiaire de type azido), conduit à un composé de formule (VII):

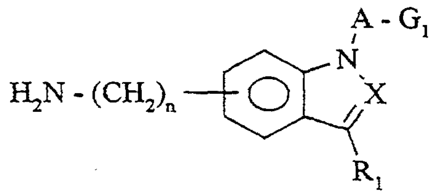

(VII)

dans laquelle n, X, A, $G_1$ et $R_1$ sont tels que définis précédemment,
qui est condensé sur la N,N-diméthylformamide azine, en milieu acide, pour conduire aux composés de formule (I),

- que l'on purifie éventuellement selon une technique classique de purification,
- dont on sépare éventuellement les énantiomères selon une technique classique de séparation,
- et que l'on transforme, le cas échéant, en leurs sels d'addition à un acide ou à une base pharmaceutiquement acceptable,
- étant entendu que lorsque Y contient un groupement hydroxy, ce dernier peut être protégé et déprotégé en fonction des réactifs utilisés.

L'invention s'étend également aux compositions pharmaceutiques renfermant comme principe actif au moins un composé de formule (I) seul ou en combinaison avec un ou plusieurs excipients ou véhicules inertes non toxiques, pharmaceutiquement acceptable. Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...
La posologie utile varie selon l'âge et le poids du patient, la nature et la sévérité de l'affection, ainsi que la voie d'administration. Celle-ci peut être nasale, rectale, parentérale ou orale. D'une manière générale, la posologie unitaire s'échelonne entre 1 et 500 mg pour un traitement en 1 à 3 prises par 24 heures.
Les exemples suivants illustrent l'invention et ne la limitent en aucune façon. Les structures des composés décrits ont été confirmées par les techniques spectroscopiques usuelles.

***EXEMPLE 1*** **: 1-(N-tertButyloxycarbonyl-pyrrolidin-3-ylméthyl)-6-([1,2,4]triazol-4-yl)indole**

*Stade a : 1-(N-tertButyloxycarbonyl-pyrrolidin-3-ylméthyl)-6-nitro-2,3-dihydroindole*

A une solution de 60,8 mmol (12,1 g) de 1-(tertbutyloxycarbonyl)-3-formylpyrrolidine, de 60,8 mmol (10 g) de 6-nitroindoline et de 60,8 mmol (3,65 g) d'acide acétique dans 100 ml de dichlorométhane, sont ajoutées, lentement à 20°C, 91,5 mmol (19,4 g) de triacétoxyborohydrure de sodium solide. Après 2 heures d'agitation, le mélange réactionnel est dilué à l'acétate d'éthyle. La phase organique est lavée à l'eau jusqu'à pH = 8, et la phase aqueuse est ensuite extraite au dichlorométhane. Après évaporation du solvant, on obtient le produit attendu.

*Stade b : 1-(N-tertButyloxycarbonyl-pyrrolidin-3-ylméthyl)-6-nitroindole*

A une solution de 63,4 mmol (20 g) du composé obtenu au stade précédent dans 1 1 de toluène, sont ajoutés 100 g de dioxyde de Manganèse. La réaction est chauffée à reflux pendant 2 heures. Après refroidissement, les sels minéraux sont filtrés et le filtrat concentré. Le résidu est purifié par chromatographie sur gel de silice en utilisant comme éluant un mélange dichlorométhane/méthanol, 99/1, pour conduire au produit attendu.

*Stade c : 6-Amino-1-(N-tertButyloxycarbonyl-pyrrolidin-3-ylméthyl)-indole*

Une solution de 28,1 mmol (9,7 g) du composé décrit au stade précédent dans 350 ml d'éthanol, en présence de 4,7 g de palladium sur charbon, est agitée pendant 15 minutes sous pression atmosphérique d'hydrogène. Le catalyseur est filtré, et le filtrat concentré pour conduire au composé attendu.

*Stade d: 1-(N-tertButyloxycarbonyl-pyrrolidin-3-ylméthyl)-6-([1,2,4]triazol-4-yl)indole*

Une solution de 24,8 mmol de composé décrit au stade précédent, de 24,6 mmol de N,N-diméthylformamide azine (obtenu selon le procédé décrit dans J. Med. Chem., 1967, 1664) et de 300 mg d'acide paratoluènesulfonique dans 100 ml de toluène est portée à reflux pendant 12 heures. Après refroidissement, la phase organique est lavée à l'eau, et la phase aqueuse est extraite au dichlorométhane. Les différentes phases organiques réunies sont concentrées, et le résidu est purifié par chromatographie sur gel de silice en utilisant comme éluant un mélange dichlorométhane/méthanol/ammoniaque, 95/5/0,5, pour conduire au composé désiré.

***EXEMPLE 2 : 1-(Diméthylaminoéthyl)-6-([1,2,4]triazol-4-yl)indole, dichlorhydrate***

Le produit attendu est obtenu selon le procédé décrit dans l'exemple 1, stade d, à partir du 6-amino-1-(2-diméthylaminoéthyl)indole décrit dans la demande WO 95/32967. Le dichlorhydrate correspondant est obtenu par action d'une solution titrée d'acide chlorhydrique dans l'éthanol.
*Point de fusion : 254-256°C*

***EXEMPLE 3 : 1-(1-Benzylpipérid-4-yl)-6-([1,2,4]triazol-4-yl)indole, dichlorhydrate***

*Stade a : 1-(1-Benzylpipérid-4-yl)-6-nitro-2,3-dihydroindole*

Le produit attendu est obtenu selon le procédé décrit dans l'exemple 1, stade a, en remplaçant la 1-(tertbutyloxycarbonyl)-3-formylpyrrolidine par la N-benzyl-4-pipéridone.

*Stade b : 1-(1-Benzylpipérid-4-yl)-6-nitroindole*

Le produit attendu est obtenu selon le procédé décrit dans l'exemple 1, stade b, à partir du composé décrit au stade précédent.

*Stade c : 6-Amino-1-(1-benzylpipérid-4-yl)indole*

Le produit attendu est obtenu selon le procédé décrit dans l'exemple 1, stade c, à partir du composé décrit au stade précédent.

*Stade d : 1-(1-Benzylpipérid-4-yl)-6-([1,2,4]triazol-4-yl)indole, dichlorhydrate*

Le produit attendu est obtenu selon le procédé décrit dans l'exemple 1, stade d, à partir du composé décrit au stade précédent, suivi d'un traitement de la base par une solution titrée d'acide chlorhydrique dans l'éthanol.

***EXEMPLE 4 : 1-[3-(4-Benzylpipérid-1-yl)propyl]-6-([1,2,4]triazol-4-yl)indole, dichlorhydrate***

*Stade a : 1-(3-Hydroxypropyl)-6-nitroindole*

A une solution de 0,11 mol de 6-nitroindole dans 400 ml de diméthylformamide sont ajoutées 0,13 mol de tertiobutylate de potassium et 0,13 mol de 3-bromopropanol. Le milieu réactionnel est chauffé à 80°C pendant 3 heures. Après refroidissement, 300 ml d'une solution de carbonate de potassium à 10 % sont ajoutés et le milieu est dilué à l'acétate d'éthyle. Après décantation, la phase aqueuse est extraite au dichlorométhane, puis les phases organiques regroupées sont concentrées et purifiées par chromatographie sur gel de silice pour conduire au produit attendu.

*Stade b : 1-(3-Bromopropyl)-6-nitroindole*

A une solution de 0,1 mol du composé décrit au stade précédent dans 300 ml de dichlorométhane, sont ajoutées 0,12 mol de tétrabromure de carbone, puis une solution de 0,12 mol de triphénylphosphine en solution dans

100 ml de dichlorométhane. Après 4 heures d'agitation à température ambiante, le milieu réactionnel est concentré et purifié par chromatographie sur gel de silice pour conduire au composé attendu.

*Stade c : 1-[3-(4-Benzylpipérid-1-yl)propyl]-6-nitroindole*

A une solution de 10 mmol du composé décrit au stade précédent dans 30 ml de diéthylcétone, sont ajoutées 12 mmol de triéthylamine et 12 mmol de 4-benzylpipéridine. Le milieu réactionnel est agité 6 heures à température ambiante. Après hydrolyse, le milieu est dilué à l'acétate d'éthyle, et extrait. Les phases organiques sont concentrées et purifiées par chromatographie sur gel de silice pour conduire au composé attendu.

*Stade d : 6-Amino-1-[3-(4-benzylpipérid-1-yl)propyl]-indole*

Le produit attendu est obtenu selon le procédé décrit dans l'exemple 1, stade c, à partir du composé décrit au stade précédent.

*Stade e : 1-[3-(4-Benzylpipérid-1-yl)propyl]-6-([1,2,4]triazol-4-yl)indole, dichlorhydrate*

Le produit attendu est obtenu selon le procédé décrit dans l'exemple 1, stade d, à partir du composé décrit au stade précédent. Le dichlorhydrate correspondant est obtenu par action d'une solution titrée d'acide chlorhydrique dans l'éthanol.

***EXEMPLE 5 : 1-{3-[4-(5-Méthoxypyrimidin-4-yl)pipérazin-1-yl]propyl}-6-([1,2,4] triazol-4-yl)indole, dichlorhydrate***

*Stade a : 1-{3-[4-(5-Méthoxypyrimidin-4-yl)pipérazin-1-yl]propyl}-6-nitroindole*

Le produit attendu est obtenu selon le procédé décrit dans l'exemple 4, stade c, en remplaçant la 4-benzylpipéridine par la 4-(5-méthoxypyrimidin-4-yl)pipérazine.

*Stade b : 6-Amino-1-{3-[4-(5-méthoxypyrimidin-4-yl)pipérazin-1-yl]propyl}-indole*

Le produit attendu est obtenu selon le procédé décrit dans l'exemple 1, stade c, à partir du composé décrit au stade précédent.

*Stade c : 1-{3-[4-(5-Méthoxypyrimidin-4-yl)pipérazin-1-yl]propyl}-6-([1,2,4]triazol-4-yl) indole, dichlorhydrate*

Le produit attendu est obtenu selon le procédé décrit dans l'exemple 1, stade d, à partir du composé décrit au stade précédent. Le chlorhydrate correspondant est obtenu par action d'une solution titrée d'acide chlorhydrique dans l'éthanol.
*Point de fusion : 130-132°C*

***EXEMPLE 6 : 1-(Diméthylaminoéthyl)-6-([1,2,4]triazol-4-yl)indazole, dichlorhydrate***

*Stade a : 1-(2-Diméthylaminoéthyl)-6-nitroindazole*

A une solution de 0,11 mol (20 g) de 6-nitroindazole dans 400 ml de tétrahydrofurane sont ajoutées 0,11 mol de tertiobutylate de potassium. Après dissolution totale, une solution de 0,24 mol (27 g) de 2-chloroéthyl-N,N-diméthylamine dans 300 ml de toluène est ajoutée. Le milieu réactionnel est chauffé à reflux pendant 3 heures. Après refroidissement, 300 ml d'une solution de carbonate de sodium à 10 % sont ajoutés. Le milieu est dilué à l'acétate d'éthyle, décanté, et la phase aqueuse est extraite au dichlorométhane. Après concentration des phases organiques, le résidu est purifié par chromatographie sur gel de silice en utilisant comme éluant un mélange dichlorométhane/méthanol, 98/2, pour conduire au composé attendu.

*Stade b : 6-Amino-1-(2-diméthylaminoéthyl)indazole*

Le produit attendu est obtenu selon le procédé décrit dans l'exemple 1, stade c, à partir du composé décrit au stade précédent.

*Stade c : 1-(2-Diméthylaminoéthyl)-6-([1,2,4]triazol-4-yl)indazole, dichlorhydrate*

Le produit attendu est obtenu selon le procédé décrit dans l'exemple 1, stade d, à partir du composé décrit au stade précédent. Le dichlorhydrate correspondant est obtenu par action d'une solution titrée d'acide chlorhydrique dans l'éthanol.

***EXEMPLE 7 : 1-{3-[4-(5-Méthoxypyrimidin-4-yl)pipérazin-1-yl]propyl}-6-([1,2,4] triazol-4-yl)indazole***

*Stade a : 1-(3-Hydroxypropyl)-6-nitroindazole*

Le produit attendu est obtenu selon le procédé décrit dans l'exemple 4, stade a, en remplaçant le 6-nitroindole par le 6-nitroindazole, et après séparation des isomères de position formés.

*Stade b : 1-(3-Bromopropyl)-6-nitroindazole*

Le produit attendu est obtenu selon le procédé décrit dans l'exemple 4, stade b, à partir du composé décrit au stade précédent.

*Stade c :* 1-{3-[4-(5-Méthoxypyrimidin-4-yl)pipérazin-1-yl]propyl]-6-nitroindazole

Le produit attendu est obtenu selon le procédé décrit dans l'exemple 4, stade c, à partir du composé décrit au stade précédent, en remplaçant la 4-benzylpipéridine par la 4-(5-méthoxypirimidin-4-yl)pipérazine.

*Stade d : 6-Amino-1-{3-[4-(5-méthoxypyrimidin-4-yl)pipérazin-1-yl]propyl}-indazole*

Le produit attendu est obtenu selon le procédé décrit dans l'exemple 1, stade c, à partir du composé décrit au stade précédent.

*Stade e : 1-{3-[4-(5-Méthoxypyrimidin-4-yl)pipérazin-1-yl]propyl}-6-([1,2,4]triazol-4-yl) indazole*

Le produit attendu est obtenu selon le procédé décrit dans l'exemple 1, stade d, à partir du composé décrit au stade précédent.

***EXEMPLE 8 : 1-(1-Méthylpipérid-4-yl)-6-([1,2,4]triazol-4-yl)indole, dichlorhydrate***

*Stade a : 1-(1-Méthylpipérid-4-yl)-6-nitro-2,3-dihydroindole*

Le produit attendu est obtenu selon le procédé décrit dans l'exemple 1, stade a, en remplaçant le 1-(tertbutyloxycarbonyl)-3-formylpyrrolidine par la N-méthyl-4-pipéridone.

*Stade b : 1-(1-Méthylpipérid-4-yl)-6-nitroindole*

Le produit attendu est obtenu selon le procédé décrit dans l'exemple 1, stade b, à partir du composé décrit au stade précédent.

*Stade c : 6-amino-1-(1-Méthylpipérid-4-yl)indole*

Le produit attendu est obtenu selon le procédé décrit dans l'exemple 1, stade c, à partir du composé décrit au stade précédent.

*Stade d : 1-(1-Méthylpipérid-4-yl)-6-([1, 2,4]triazol-4-yl)indole, dichlorhydrate*

Le produit attendu est obtenu selon le procédé décrit dans l'exemple 1, stade d, à partir du composé décrit au stade précédent.

La base obtenue est alors salifiée par une solution titrée d'acide chlorhydrique dans l'éthanol.
*Point de fusion : 195-198°C*

***EXEMPLE 9** : 1-{3-[4-(2-Phényléthyl)pipérazin-1-yl]propyl}-6-([1,2,4]triazol-4-yl) indole, dichlorhydrate*

*Stade a : 1-{3-[4-(2-Phényléthyl)pipérazine-1-yl]propyl}-6-nitroindole*

Le produit attendu est obtenu selon le procédé décrit dans l'exemple 4, stade c, en remplaçant la 4-benzylpipéridine par la N-(2-phényléthyl)pipérazine.

*Stade b : 6-Amino-1-{3-[4-(2-phényléthyl)pipérazine-1-yl]propyl}indole*

Le produit attendu est obtenu selon le procédé décrit dans l'exemple 1, stade c, à partir du composé décrit au stade précédent.

*Stade c : 1-{3-[4-(2-Phényléthyl)pipérazine-1-yl]propyl}-6-([1,2,4]triazol-4-yl) indole, dichlorhydrate*

Le produit attendu est obtenu selon le procédé décrit dans l'exemple 1, stade d, à partir du composé décrit au stade précédent.
La base obtenue est alors salifiée par une solution titrée d'acide chlorhydrique dans l'éthanol.
*Point de fusion : >270°C*

***EXEMPLE 10** : 1-(Pyrrolidin-3-ylméthyl)-6-([1,2,4]triazol-4-yl)indole dichlorhydrate*

Une solution de 30 mmol du composé décrit dans l'exemple 1 dans 300 ml d'acétate d'éthyle est traitée pendant 4 minutes à -10°C par un courant d'acide chlorhydrique. Le précipité obtenu est filtré et rinçé à l'éther pour conduire au produit du titre.
*Point de fusion : 206°C*

***EXEMPLE 11** : 1-[(N-Ethylpyrrolidin-3-yl)méthyl]-6-([1,2,4]triazol-4-yl)indole dichlorhydrate*

*Stade a : 1-[(Pyrrolidin-3-yl)méthyl]-6-nitroindole*

Le produit attendu est obtenu selon le procédé décrit dans l'exemple 10, en utilisant comme produit de départ le composé décrit dans l'exemple 1, stade b.

*Stade b : 1-[(N-Ethylpyrrolidin-3-yl)méthyl]-6-nitroindole*

Le produit attendu est obtenu selon le procédé décrit dans l'exemple 1, stade a, en remplaçant la 1-(tertbutyloxycarbonyl)-3-formylpyrrolidine par l'acétaldéhyde, et en utilisant comme produit de départ le composé décrit au stade précédent.

*Stade c : 1-[(N-Ethylpyrrolidin-3-yl)méthyl]-6-([1,2,4]triazol-4-yl)indole dichlorhydrate*

Le produit attendu est obtenu selon le procédé décrit dans l'exemple 1, stades c et d, en utilisant comme produit de départ le composé décrit au stade précédent. Le dichlorhydrate correspondant est obtenu par action d'une solution titrée d'acide chlorhydrique dans l'éthanol.

***EXEMPLE 12** : 1-{[N-(Cyclohexylméthyl)pyrrolidin-3-yl]méthyl}-6-([1,2,4]triazol-4-yl) indole, dichlorhydrate*

*Stade a : 1-{[N-(Cyclohexylméthyl)pyrrolidin-3-yl]méthyl}-6-nitroindole*

Le produit attendu est obtenu selon le procédé décrit dans l'exemple 1, stade a, en remplaçant la 1-(tertbutyloxycarbonyl)-3-formylpyrrolidine par le cyclohexane-carboxaidéhyde et en utilisant comme produit de départ le composé décrit au stade a de l'exemple 11.

*Stade b : 1-{[N-(Cyclohexylméthyl)pyrrolidin-3-yl]méthyl}-6-([1,2,4]triazol-4-yl)indole, dichlorhydrate*

Le produit attendu est obtenu selon le procédé décrit dans l'exemple 1, stades c et d, en utilisant comme produit de départ le composé décrit au stade précédent. Le dichlorhydrate correspondant est obtenu par action d'une solution titrée d'acide chlorhydrique dans l'éthanol.

***EXEMPLE 13* : 1-{[N-(2-Furylméthyl)pyrrolidin-3-yl]méthyl}-6-([1,2,4]triazol-4-yl) indole, dichlorhydrate**

*Stade a: 1-{[N-(Furylméthyl)pyrrolidin-3-yl]méthyl}-6-nitroindole*

Le produit attendu est obtenu selon le procédé décrit dans l'exemple 1, stade a, en remplaçant la 1-(tertbutyloxycarbonyl)-3-formylpyrrolidine par le 2-furfuraldéhyde et en utilisant comme produit de départ le composé décrit au stade a de l'exemple 11.

*Stade b : 1-{[N-(2-Furylméthyl)pyrrolidin-3-yl]méthyl}-6-([1,2,4]triazol-4-yl)indole, dichlorhydrate*

Le produit attendu est obtenu selon le procédé décrit dans l'exemple 1, stades c et d, en utilisant comme produit de départ le composé décrit au stade précédent. Le dichlorhydrate correspondant est obtenu par action d'une solution titrée d'acide chlorhydrique dans l'éthanol.

***EXEMPLE 14* : 1-{[N-(2-Pyridylméthyl)pyrrolidin-3-yl]méthyl}-6-([1,2,4]triazol-4-yl) indole, dichlorhydrate**

*Stade a : 6-Nitro-1-{[N-(2-pyridylméthyl)pyrrolidin-3-yl]méthyl}indole*

Le produit attendu est obtenu selon le procédé décrit dans l'exemple 1, stade a, en remplaçant la 1-(tertbutyloxycarbonyl)-3-formylpyrrolidine par le 2-pyridinecarboxaldéhyde et en utilisant comme produit de départ le composé décrit au stade a de l'exemple 11.

*Stade b : 1-{[N-(2-Pyridylméthyl)pyrrolidin-3-yl]méthyl}-6-([1,2,4]triazol-4-yl)indole, dichlorhydrate*

Le produit attendu est obtenu selon le procédé décrit dans l'exemple 1, stades c et d, en utilisant comme produit de départ le composé décrit au stade précédent. Le dichlorhydrate correspondant est obtenu par action d'une solution titrée d'acide chlorhydrique dans l'éthanol.

***EXEMPLE 15* : 1-(4-Pipéridyl)-6-([1,2,4]triazol-4-yl)indole, dichlorhydrate**

*Stade a : 6-Nitro-1-(1-carbéthoxy-4-pipéridyl)indole*

Le produit attendu est obtenu selon le procédé décrit dans l'exemple 1, stade a, en remplaçant la 1-(tertbutyloxycarbonyl)-3-formylpyrrolidine par la N-carbéthoxy-4-pipéridone.

*Stade b : 1-(1-Carbéthoxy-4-pipéridyl)-6-([1,2,4]triazol-4-yl)indole*

Le produit attendu est obtenu selon le procédé décrit dans l'exemple 1, stades c et d, en utilisant comme produit de départ le composé décrit au stade précédent.

*Stade c : 1-(4-Pipéridyl)-6-([1,2,4]triazol-4-yl)indole, dichlorhydrate*

Le produit attendu est obtenu selon le procédé décrit dans l'exemple 10, en utilisant comme produit de départ le composé décrit au stade précédent, et une solution aqueuse d'acide chlorhydrique.

***EXEMPLE 16* : 1-[1-(2-Pyridylméthyl)pipérid-4-yl]-6-([1,2,4]triazol-4-yl)indole, dichlorhydrate**

*Stade a : 6-Nitro-1-[1-(2-Pyridylméthyl)-4-pipérid-4-yl]indole*

Le produit attendu est obtenu selon le procédé décrit dans l'exemple 1, stade a, en remplaçant la 1-(tertbutyloxycarbonyl)-3-formylpyrrolidine par la 2-pyridinecarboxaldéhyde, et en utilisant comme produit de départ le composé décrit dans l'exemple 15, stade a.

*Stade b : 1-[1-(2-Pyridylméthyl)pipérid-4-yl]-6-([1,2,4]triazol-4-yl)indole, dichlorhydrate*

Le produit attendu est obtenu selon le procédé décrit dans l'exemple 1, stades c et d, en utilisant comme produit de départ le composé décrit au stade précédent. Le dichlorhydrate correspondant est obtenu par action d'une solution titrée d'acide chlorhydrique dans l'éthanol.

***EXEMPLE 17**: 1-[1-(2-Furylméthyl)pipérid-4-yl]-6-([1,2,4]triazol-4-yl)indole, dichlorhydrate*

*Stade a : 1-[1-(2-Furylméthyl)pipérid-4-yl]-6-nitroindole*

Le produit attendu est obtenu selon le procédé décrit dans l'exemple 1, stade a, en remplaçant la 1-(tertbutyloxycarbonyl)-3-fôrmylpyrrolidine par la 2-furfuraldéhyde, et en utilisant comme produit de départ le composé décrit dans l'exemple 15, stade a.

*Stade b : 1-[1-(2-Furylméthyl)pipérid-4-yl]-6-([1,2,4]triazol-4-yl)indole, dichlorhydrate*

Le produit attendu est obtenu selon le procédé décrit dans l'exemple 1, stades c et d, en utilisant comme produit de départ le composé décrit au stade précédent. Le dichlorhydrate correspondant est obtenu par action d'une solution titrée d'acide chlorhydrique dans l'éthanol.

***EXEMPLE 18** : 1-[1-(3-Phénylpropyl)pipérid-4-yl)-6-([1,2,4]triazol-4-yl)indole, dichlorhydrate*

*Stade a : 6-Nitro-1-[1-(3-phényl-2-propényl)pipérid-4-yl)indole*

Le produit attendu est obtenu selon le procédé décrit dans l'exemple 1, stade a, en remplaçant la 1-(tertbutyloxycarbonyl)-3-formylpyrrolidine par l'aldéhyde cinnamique et en utilisant comme produit de départ le composé décrit dans l'exemple 15, stade a.

*Stade b : 1-[1-(3-Phénylpropyl)pipérid-4-yl]-6-([1,2,4,]triazol-4-yl)indole, dichlorhydrate*

Le produit attendu est obtenu selon le procédé décrit dans l'exemple 1, stades c et d, en utilisant comme produit de départ le composé décrit au stade précédent. Le dichlorhydrate correspondant est obtenu par action d'une solution titrée d'acide chlorhydrique dans l'éthanol.

***EXEMPLE 19** : 1-[1-(Cyclohexylméthyl)pipérid-4-yl]-6-([1,2,4]triazol-4-yl)indole, dichlorhydrate*

*Stade a : 1-[1- (Cyclohexylméthyl)pipérid-4-yl]-6-nitroindole*

Le produit attendu est obtenu selon le procédé décrit dans l'exemple 1, stade a, en remplaçant la 1-(tertbutyloxycarbonyl)-3-formylpyrrolidine par le cyclohexanecarboxaldéhyde, et en utilisant comme produit de départ le composé décrit dans l'exemple 15, stade a.

*Stade b : 1-[1-(Cyclohexylméthyl)pipérid-4-yl]-6-([1,2,4]triazol-4-yl)indole, dichlorhydrate*

Le produit attendu est obtenu selon le procédé décrit dans l'exemple 1, stades c et d, en utilisant comme produit de départ le composé décrit au stade précédent. Le dichlorhydrate correspondant est obtenu par action d'une solution titrée d'acide chlorhydrique dans l'éthanol.

***EXEMPLE 20** : 1-[3-(2-Méthylpipérid-1-yl)propyl]-6-([1,2,4]triazol-4-yl)indole, dichlorhydrate*

Le produit attendu est obtenu selon le procédé décrit dans l'exemple 4, en remplaçant, au stade c, la 4-benzylpipéridine par la 2-méthylpipéridine.

***EXEMPLE 21** : 1-[3-(4-Méthylpipérid-1-yl)propyl]-6-([1,2,4]triazol-4-yl)indole, dichlorhydrate*

Le produit attendu est obtenu selon le procédé décrit dans l'exemple 4, en remplaçant, au stade c, la 4-benzylpipéridine par la 4-méthylpipéridine.

***EXEMPLE 22** : 1-[3-(2,6-Diméthylpipérid-1-yl)propyl]-6-([1,2,4]triazol-4-yl)indole, dichlorhydrate*

Le produit attendu est obtenu selon le procédé décrit dans l'exemple 4, en remplaçant, au stade c, la 4-benzylpipéridine par la 2,6-diméthylpipéridine.

***EXEMPLE 23** : 1-[3-(3,5-Diméthylpipérid-1-yl)propyl]-6-([1,2,4]triazol-4-yl)indole, dichlorhydrate*

Le produit attendu est obtenu selon le procédé décrit dans l'exemple 4, en remplaçant, au stade c, la 4-benzylpipéridine par la 3,5-diméthylpipéridine.

***EXEMPLE 24** : 1-[2-(Benzylamino)éthyl]-6-([1,2,4]triazol-4-yl)indole, dichlorhydrate*

Le produit attendu est obtenu selon le procédé décrit dans l'exemple 4, en remplaçant, au stade a, le 3-bromopropanol par le 2-bromoéthanol, et au stade c, la 4-benzylpipéridine par la benzylamine.

***EXEMPLE 25** : 1-[3-(Benzylamino)propyl]-6-([1,2,4]triazol-4-yl)indole, dichlorhydrate*

Le produit attendu est obtenu selon le procédé décrit dans l'exemple 4, en remplaçant, au stade c, la 4-benzylpipéridine par la benzylamine.

***EXEMPLE 26** : 1-[3-(Phénylamino)propyl]-6-([1,2,4]triazol-4-yl)indole, dichlorhydrate*

Le produit attendu est obtenu selon le procédé décrit dans l'exemple 4, en remplaçant, au stade c, la 4-benzylpipéridine par l'aniline.

***EXEMPLE 27** : 1-[3-(Cyclohexylamino)propyl]-6-([1,2,4]triazol-4-yl)indole, dichlorhydrate*

Le produit attendu est obtenu selon le procédé décrit dans l'exemple 4, en remplaçant, au stade c, la 4-benzylpipéridine par la cyclohexylamine.

***EXEMPLE 28** : 1-[3-(1-Pipérazinyl)propyl]-6-([1,2,4]triazol-4-yl)indole, dichlorhydrate*

Le produit attendu est obtenu selon le procédé décrit dans l'exemple 4, en remplaçant, au stade c, la 4-benzylpipéridine par la pipérazine, en large excès.

***EXEMPLE 29** : 1-[3-(4-Phénylpipérazin-1-yl)propyl]-6-([1,2,4]triazol-4-yl)indole, dichlorhydrate*

Le produit attendu est obtenu selon le procédé décrit dans l'exemple 4, en remplaçant, au stade c, la 4-benzylpipéridine par la 4-phénylpipérazine.

***EXEMPLE 30** : 1-{3-[4-(2-Pyridyl)pipérazin-1-yl]propyl}-6-([1,2,4]triazol-4-yl)indole, dichlorhydrate*

Le produit attendu est obtenu selon le procédé décrit dans l'exemple 4, en remplaçant, au stade c, la 4-benzylpipéridine par la 1-(2-pyridyl)pipérazine.

***EXEMPLE 31** : 1-[3-(4-Méthylpipérazin-1-yl)propyl]-6-([1,2,4]triazol-4-yl)indole, dichlorhydrate*

Le produit attendu est obtenu selon le procédé décrit dans l'exemple 4, en remplaçant, au stade c, la 4-benzylpipéridine par la 1-méthylpipérazine.

***EXEMPLE 32** : 1-[3-(4-Cyclohexylpipérazin-1-yl)propyl]-6-([1,2,4]triazol-4-yl)indole, dichlorhydrate*

Le produit attendu est obtenu selon le procédé décrit dans l'exemple 4, en remplaçant, au stade c, la 4-benzylpipéridine par la cyclohexylpipérazine.

***EXEMPLE 33** : 1-{3-[4-(2-Pyridylméthyl)pipérazin-1-yl]propyl}-6-([1,2,4]triazol-4-yl) indole, dichlorhydrate*

*Stade a : 6-Nitro-1-{3-[4-(2-pyridylméthyl)pipérazin-1-yl]propyl}indole*

Le produit attendu est obtenu selon le procédé décrit dans l'exemple 1, stade a, en remplaçant la 1-(tertbutyloxycarbonyl)-3-formylpyrrolidine par le 2-pyridinecarboxaldéhyde et en utilisant comme produit de départ le 6-nitro-1-[(1-pipérazinyl)propyl]indole (isolé au cours du procédé de préparation du composé décrit dans l'exemple 28.

*Stade b : 1-{3-[4-(2-Pyridylméthyl)pipérazin-1-yl]propyl}-6-([1,2,4]triazol-4-yl)indole, dichlorhydrate*

Le produit attendu est obtenu selon le procédé décrit dans l'exemple 1, stades c et d, en utilisant comme produit de départ le composé décrit au stade précédent. Le dichlorhydrate correspondant est obtenu par action d'une solution titrée d'acide chlorhydrique dans l'éthanol.

***EXEMPLE 34 : 1-{3-[4-(3-Trifluorométhylphényl)pipérazin-1-yl)propyl}-6-([1,2,4] triazol-4-yl)indole, dichlorhydrate***

Le produit attendu est obtenu selon le procédé décrit dans l'exemple 4, en remplaçant, au stade c, la 4-benzylpipéridine par la 1-(3-trifluorométhylphényl)pipérazine.

***EXEMPLE 35 : 1-[N-(tertButyloxycarbonylpyrrolidin-3-yl)méthyl]-6-([1,2,4]triazol-4-yl) indazole***

Le produit attendu est obtenu selon le procédé décrit dans l'exemple 1, stades a, c et d, en remplaçant la 6-nitroindoline par le 6-nitroindazole.

***EXEMPLE 36 : 1-(3-Pyrrolidinylméthyl)-6-([1,2,4]triazol-4-yl)indazole, dichlorhydrate***

Le produit attendu est obtenu selon le procédé décrit dans l'exemple 10, en utilisant comme produit de départ le composé décrit dans l'exemple 35.

***EXEMPLE 37 : 1-{[N-(Cyclohexylméthyl)pyrrolidin-3-yl]méthyl}-6-([1,2,4]triazol-4-yl) indazole, dichlorhydrate***

*Stade a: 6-Nitro-1-(3-pyrrolidinylméthyl)indazole*

Le produit attendu est obtenu selon le procédé décrit dans l'exemple 10, en utilisant comme produit de départ le 1-[N-(tertButyloxycarbonylpyrrolidin-3-yl)méthyl]-6-nitroindazole, isolé au cours du procédé de préparation du composé décrit dans l'exemple 35.

*Stade b : 1-{[N-(Cyclohexylméthyl)pyrrolidin-3-yl]méthyl}-6-nitro indazole*

Le produit attendu est obtenu selon le procédé décrit dans l'exemple 1, stade a, en remplaçant la 1-(tertbutyloxycarbonyl)-3-formylpyrrolidine par le cyclohexane-carboxaldéhyde et en utilisant comme produit de départ le composé décrit au stade précédent.

*Stade c : 1-{[N-(Cyclohexylméthyl)pyrrolidin-3-yl]méthyl}-6-([1,2,4]triazol-4-yl) indazole, dichlorhydrate*

Le produit attendu est obtenu selon le procédé décrit dans l'exemple 1, stades c et d, en utilisant comme produit de départ le composé décrit au stade précédent.
Le dichlorhydrate correspondant est obtenu par action d'une solution titrée d'acide chlorhydrique dans l'éthanol.

***EXEMPLE 38 : 1-{[N-(2-Pyridylméthyl)pyrrolidin-3-yl]méthyl}-6-([1,2,4]triazol-4-yl) indazole, dichlorhydrate***

*Stade a : 6-Nitro-1-{[N-(2-pyridylméthyl)pyrrolidin-3-yl]méthyl}indazole*

Le produit attendu est obtenu selon le procédé décrit dans l'exemple 1, stade a, en remplaçant la 1-(tertbutyloxycarbonyl)-3-formylpyrrolidine par le 2-pyridinecarboxaldéhyde et en utilisant comme produit de départ le composé décrit dans l'exemple 37, stade a.

*Stade b : 1-{[N-(2-Pyridylméthyl)pyrrolidin-3-yl]méthyl}-6-([1,2,4]triazol-4-yl)indazole. dichlorhydrate*

Le produit attendu est obtenu selon le procédé décrit dans l'exemple 1, stades c et d, en utilisant comme produit de départ le composé décrit au stade précédent.
Le dichlorhydrate correspondant est obtenu par action d'une solution titrée d'acide chlorhydrique dans l'éthanol.

***EXEMPLE 39** : **1-(4-Pipéridyl)-6-([1,2,4]triazol-4-yl)indazole, dichlorhydrate***

*Stade a : 6-Nitro-1-(1-carbéthoxy-4-pipéridyl)indazole*

Le produit attendu est obtenu selon le procédé décrit dans l'exemple 1, stade a, en remplaçant la 1-(tertbutyloxycarbonyl)-3-formylpyrrolidine par la N-carbéthoxy-4-pipéridone, et en utilisant comme produit de départ le 6-nitroindazole.

*Stade b : 1-(1-Carbéthoxy-4-pipéridyl)-6-([1,2,4]triazol-4-yl)indazole*

Le produit attendu est obtenu selon le procédé décrit dans l'exemple 1, stades c et d, en utilisant comme produit de départ le composé décrit au stade précédent.

*Stade c : 1-(4-Pipéridyl)-6-([1,2,4]triazol-4-yl)indazole, dichlorhydrate*

Le produit attendu est obtenu selon le procédé décrit dans l'exemple 10, en utilisant comme produit de départ le composé décrit au stade précédent, et une solution aqueuse d'acide chlorhydrique.

***EXEMPLE 40** : **1-(N-Benzylpipérid-4-yl)-6-([1,2,4]triazol-4-yl)indazole, dichlorhydrate***

*Stade a : 1-(N-Benzylpipérid-4-yl)-6-nitroindazole*

Le produit attendu est obtenu selon le procédé décrit dans l'exemple 1, stade a, en remplaçant la 1-(tertbutyloxycarbonyl)-3-formylpyrrolidine par la N-benzyl-4-pipéridone, et en utilisant comme produit de départ le 6-nitroindazole.

*Stade b : 1-(N-Benzylpipérid-4-yl)-6-([1,2,4]triazol-4-yl)indazole, dichlorhydrate*

Le produit attendu est obtenu selon le procédé décrit dans l'exemple 1, stades c et d, en utilisant comme produit de départ le composé décrit au stade précédent.

Le dichlorhydrate correspondant est obtenu par action d'une solution titrée d'acide chlorhydrique dans l'éthanol.

***EXEMPLE 41** : **1-[N-(2-Furylméthyl)pipérid-4-yl]-6-([1,2,4]triazol-4-yl)indazole, dichlorhydrate***

*Stade a : 1-[N-(2-Furylméthyl)pipérid-4-yl]-6-nitroindazole*

Le produit attendu est obtenu selon le procédé décrit dans l'exemple 1, stade a, en remplaçant la 1-(tertbutyloxycarbonyl)-3-formylpyrrolidine par le 2-furfuraldéhyde, et en utilisant comme produit de départ le composé décrit dans l'exemple 39, stade a.

*Stade b : 1-[N-(2-Furylméthyl)pipérid-4-yl]-6-([1,2,4]triazol-4-yl)indazole, dichlorhydrate*

Le produit attendu est obtenu selon le procédé décrit dans l'exemple 1, stades c et d, en utilisant comme produit de départ le composé décrit au stade précédent.

Le dichlorhydrate correspondant est obtenu par action d'une solution titrée d'acide chlorhydrique dans l'éthanol.

***EXEMPLE 42** : **1-(N-méthylpipéridin-4-yl)-6-([1,2,4]triazol-4-yl)indazole, dichlorhydrate***

*Stade a : 1-(N-Méthylpipérid-4-yl)-6-nitroindazole*

Le produit attendu est obtenu selon le procédé décrit dans l'exemple 1, stade a, en remplaçant la 1-(tertbutyloxycarbonyl)-3-formylpyrrolidine par la N-méthyl-4-pipéridone.

*Stade b : 1-(N-méthylpipérid-4-yl)-6-([1,2,4]triazol-4-yl)indazole, dichlorhydrate*

Le produit attendu est obtenu selon le procédé décrit dans l'exemple 1, stades c et d, en utilisant comme

produit de départ le composé décrit au stade précédent.

Le dichlorhydrate correspondant est obtenu par action d'une solution titrée d'acide chlorhydrique dans l'éthanol.

***<u>EXEMPLE 43</u> : 1-[3-(Benzylamino)propyl]-6-([1,2,4]triazol-4-yl)indazole, dichlorhydrate***

Le produit attendu est obtenu selon le procédé décrit dans l'exemple 4, en utilisant comme produit de départ le 6-nitroindazole et en remplaçant, au stade c, la 4-benzylpipéridine par la benzylamine et en utilisant comme agent réducteur, au stade d, l'hydrazine en présence de nickel.

***<u>EXEMPLE 44</u> : 1-[3-(1-Pipérazinyl)propyl]-6-([1,2,4]triazol-4-yl)indazole, dichlorhydrate***

Le produit attendu est obtenu selon le procédé décrit dans l'exemple 4, en utilisant comme produit de départ le 6-nitroindazole et en remplaçant, au stade c, la 4-benzylpipéridine par la pipérazine en large excès.

***<u>EXEMPLE 45</u> : 1-{3-[4-(2-Phényléthyl)pipérazin-1-yl]propyl}-6-([1,2,4]triazol-4-yl) indazole, dichlorhydrate***

Le produit attendu est obtenu selon le procédé décrit dans l'exemple 4, en utilisant comme produit de départ le 6-nitroindazole et en remplaçant, au stade c, la 4-benzylpipéridine par la 4-(2-phényléthyl)pipérazine.

***<u>EXEMPLE 46</u> : 1-{3-[4-(3-Trifluorométhylphényl)pipérazin-1-yl]propyl}-6-([1,2,4] triazol-4-yl)indazole, dichlorhydrate***

Le produit attendu est obtenu selon le procédé décrit dans l'exemple 4, en utilisant comme produit de départ le 6-nitroindazole et en remplaçant, au stade c, la 4-benzylpipéridine par la 1-(3-trifluorométhylphényl)pipérazine.

***<u>EXEMPLE 47</u> : 1-[3-(4-Méthylpipérazin-1-yl)propyl]-6-([1,2,4]triazol-4-yl)indazole, dichlorhydrate***

Le produit attendu est obtenu selon le procédé décrit dans l'exemple 4, en utilisant comme produit de départ le 6-nitroindazole et en remplaçant, au stade c, la 4-benzylpipéridine par la 1-méthylpipérazine.

***<u>EXEMPLE 48</u> : 1-[3-(4-Cyclohexylpipérazin-1-yl)propyl]-6-([1,2,4]triazol-4-yl)indazole, dichlorhydrate***

Le produit attendu est obtenu selon le procédé décrit dans l'exemple 4, en utilisant comme produit de départ le 6-nitroindazole et en remplaçant, au stade c, la 4-benzylpipéridine par la cyclohexylpipérazine.

Les composés des exemples 49 à 56 sont obtenus selon les procédés décrits dans les exemples précédents, en utilisant comme produit de départ le 3-méthyl-6-nitroindole.

***EXEMPLE 49* : 1-(Diméthylaminoéthyl)-3-méthyl-6-([1,2,4]triazol-4-yl)indole, dichlorhydrate**

***EXEMPLE 50* : 3-Méthyl-1-[(pyrrolidin-3-yl)méthyl]-6-([1,2,4]triazol-4-yl)indole, dichlorhydrate**

***EXEMPLE 51* : 3-Méthyl-1-(1-méthylpipérid-4-yl)-6-([1,2,4]triazol-4-yl)indole, dichlorhydrate**

***EXEMPLE 52* : 1-(1-Benzylpipérid-4-yl)-3-méthyl-6-([1,2,4]triazol-4-yl)indole, dichlorhydrate**

***EXEMPLE 53* : 1-[3-(4-Benzylpipérid-1-yl)propyl]-3-méthyl-6-([1,2,4]triazol-4-yl) indole, dichlorhydrate**

***EXEMPLE 54* : 1-{3-[4-(5-Méthoxypyrimidin-4-yl)pipérazin-1-yl]propyl}-3-méthyl-6-([1,2,4]triazol-4-yl)indole, dichlorhydrate**

***EXEMPLE 55* : 3-Méthyl-1-{3-[4-(2-phényléthyl)pipérazin-1-yl]propyl}-6-([1,2,4]triazol-4-yl)indole, dichlorhydrate**

***EXEMPLE 56* : 1-[3-(4-Méthylpipérazin-1-yl)propyl]-3-méthyl-6-([1,2,4]triazol-4-yl) indole, dichlorhydrate**

***EXEMPLE 57* : 1-(1-Benzylpipérid-4-yl)-6-([1,2,4]triazol-4-ylméthyl)indole, dichlorhydrate**

*Stade a : 1-(l-Benzylpipérid-4-yl)-6-hydroxyméthylindole*

Le produit attendu est obtenu selon le procédé décrit dans l'exemple 1, stade a, en remplaçant la 1-(tertbutyloxycarbonyl)-3-formylpyrrolidine par la N-benzylpipéridone et en utilisant comme produit de départ le 6-hydroxyméthylindole (préparé à partir de l'acide carboxylique correspondant.

*Stade b : Méthanesulfonate de [1-(1-henzylpipérid-4-yl)-6-indolyl]méthyle*

A une solution de 12 mmol (3,85 g) du composé décrit au stade précédent dans 50 ml de dichlorométhane, sont ajoutées 14,5 mmol (2 ml) de triéthylamine et, goutte à goutte, 14,5 mmol (1,12 ml) de chlorure de mésyle. Le milieu réactionnel est agité à température ambiante pendant 5 heures. Après hydrolyse, et extraction au dichlorométhane, la phase organique est lavée par une solution aqueuse d'acide chlorhydrique 0,1 N, séchée et concentrée pour conduire au composé attendu.

*Stade c : 6-(Azidométhyl)-1-(1-benzylpipérid-4-yl)indole*

A une solution de 10,8 mmol du composé décrit au stade précédent dans 20 ml de diméthylsulfoxyde, sont ajoutées 21,6 mmol d'azidure de sodium. La réaction agitée à température ambiante pendant 1 heure. Après hydrolyse, le milieu réactionnel est extrait à l'acétate d'éthyle et la phase organique est séchée et concentrée pour conduire au composé attendu.

*Stade d : 6-Aminométhyl-1-(1-benzylpipérid-4-yl)indole*

Le produit attendu est obtenu selon le procédé décrit dans l'exemple 1, stade c, en utilisant comme produit de départ le composé décrit au stade précédent.

*Stade e : 1-(1-Benzylpipérid-4-yl)-6-([1,2,4]triazol-4-ylméthyl)indole, dichlorhydrate*

Le produit attendu est obtenu selon le procédé décrit dans l'exemple 1, stade d, en utilisant comme produit de départ le composé décrit au stade précédent.
*Point de fusion :* > 270°C (déc.)
De la même façon les composés des exemples 58 à 65 sont obtenus.

***EXEMPLE 58*** ***: 1-(1-Méthylpipérid-4-yl)-6-([1,2,4]triazol-4-ylméthyl)indole, dichlorhydrate***

***EXEMPLE 59*** ***: 1-[3-(4-Benzylpipérid-1-yl)propyl]-6-([1,2,4]triazol-4-ylméthyl)indole, dichlorhydrate***

***EXEMPLE 60*** ***: 1-{3-[4-(2-Phényléthyl)pipérazin-1-yl]propyl}-6-([1,2,4]triazol-4-ylméthyl)indole, dichlorhydrate***

***EXEMPLE 61*** ***: 1-{3-[4-(5-Méthoxypyrimidin-4-yl)pipérazin-1-yl]propyl}-6-([1,2,4] triazol-4-ylméthyl)indole, dichlorhydrate***

***EXEMPLE 62*** ***: 1-{[N-(2-Pyridylméthyl)pyrrolidin-3-yl]méthyl}-6-([1,2,4]triazol-4-ylméthyl)indole, dichlorhydrate***

***EXEMPLE 63*** ***: 1-(Diméthylaminoéthyl)-6-([1,2,4]triazol-4-ylméthyl)indazole, dichlorhydrate***

***EXEMPLE 64*** ***: 1-{3-[4-(2-Phényléthyl)pipérazin-1-yl]propyl}-6-([1,2,4]triazol-4-ylméthyl)indazole, dichlorhydrate***

***EXEMPLE 65*** ***: 1-{3-[4-(5-Méthoxypyrimidin-4-yl)pipérazin-1-yl]propyl}-6-([1,2,4] triazol-4-ylméthyl)indazole, dichlorhydrate***

En utilisant des procédés similaires à ceux décrits dans les exemples précédents, et en remplaçant les substrats 6-nitroindole et 6-nitroindazole par les 5-nitroindole et 5-nitroindazole, les composés des exemples 66 à 79 sont obtenus.

***EXEMPLE 66*** ***: 1-(Diméthylaminoéthyl)-5-([1,2,4]triazol-4-yl)indole, dichlorhydrate***

***EXEMPLE 67*** ***: 1-(Pyrrolidin-3-ylméthyl)-5-([1,2,4]triazol-4-yl)indole, dichlorhydrate***

***EXEMPLE 68*** ***: 1-(1-Méthylpipérid-4-yl)-5-([1,2,4]triazol-4-yl)indole, dichlorhydrate***

***EXEMPLE 69*** ***: 1-[1-(2-Pyridylméthyl)pipérid-4-yl]-5-([1,2,4]triazol-4-yl)indole, dichlorhydrate***

***EXEMPLE 70*** ***: 1-(1-Benzylpipérid-4-yl)-5-([1,2,4]triazol-4-yl)indole, dichlorhydrate***

***EXEMPLE 71*** ***: 1-[3-(4-Benzylpipérid-1-yl)propyl]-5-([1,2,4]triazol-4-yl)indole, dichlorhydrate***

***EXEMPLE 72*** ***: 1-{3-[4-(5-Méthoxypyrimidin-4-yl)pipérazin-1-yl]propyl}-5-([1,2,4] triazol-4-yl)indole, dichlorhydrate***

***EXEMPLE 73*** ***: 1-{3-[4-(2-Phényléthyl)pipérazin-1-yl]propyl}-5-([1,2,4]triazol-4-yl) indole, dichlorhydrate***

***EXEMPLE 74*** ***: 1-{3-[4-(3-Trifluorométhylphényl)pipérazin-1-yl]propyl}-5-([1,2,4] triazol-4-yl)indole, dichlorhydrate***

***EXEMPLE 75*** ***: 1-(Diméthylaminoéthyl)-5-([1,2,4]triazol-4-yl)indazole, dichlorhydrate***

***EXEMPLE 76*** ***: 1-(1-Méthylpipérid-4-yl)-5-([1,2,4]triazol-4-yl)indazole, dichlorhydrate***

***EXEMPLE 77*** ***: 1-[3-(4-Benzylpipérid-1-yl)propyl]-5-([1,2,4]triazol-4-yl)indazole, dichlorhydrate***

***EXEMPLE 78*** ***: 1-{3-[4-(2-Phényléthyl)pipérazin-1-yl]propyl}-5-([1,2,4]triazol-4-yl) indazole, dichlorhydrate***

***EXEMPLE 79*** ***: 1-{3-[4-(5-Méthoxypyrimidin-4-yl)pipérazin-1-yl]propyl}-5-([1,2,4] triazol-4-yl)indazole, dichlorhydrate***

De la même façon, en utilisant comme produit de départ le 4-nitroindole, les composés des exemples 80 à 85 sont obtenus.

***EXEMPLE 80* : 1-(Diméthylaminoéthyl)-4-([1,2,4]triazol-4-yl)indole, dichlorhydrate**

***EXEMPLE 81* : 1-(Pyrrolidin-3-ylméthyl)-4-([1,2,4]triazol-4-yl)indole, dichlorhydrate**

***EXEMPLE 82* : 1-(1-Méthylpipéridin-4-yl)-4-([1,2,4]triazol-4-yl)indole, dichlorhydrate**

***EXEMPLE 83* : 1-[3-(4-Benzylpipérid-1-yl)propyl]-4-([1,2,4]triazol-4-yl)indole, dichlorhydrate**

***EXEMPLE 84* : 1-{3-[4-(5-Méthoxypyrimidin-4-yl)pipérazin-1-yl]propyl}-4-([1,2,4] triazol-4-yl)indole, dichlorhydrate**

***EXEMPLE 85* : 1-{3-[4-(2-Phényléthyl)pipérazin-1-yl]propyl}-4-([1,2,4]triazol-4-yl) indole, dichlorhydrate**

De la même façon, en utilisant comme produit de départ le 7-nitroindazole, les composés des exemples 86 à 90 sont obtenus.

***EXEMPLE 86* : 1-(Diméthylaminoéthyl)-7-([1,2,4]triazol-4-yl)indazole, dichlorhydrate**

***EXEMPLE 87* : 1-(1-Méthylpipérid-4-yl)-7-([1,2,4]triazol-4-yl)indazole, dichlorhydrate**

***EXEMPLE 88* : 1-[3-(4-Benzylpipérid-1-yl)propyl]-7-([1,2,4]triazol-4-yl)indazole, dichlorhydrate**

***EXEMPLE 89* : 1-{3-[4-(2-Phénylethyl)pipérazin-1-yl]propyl}-7-([1,2,4]triazol-4-yl) indazole, dichlorhydrate**

***EXEMPLE 90* : 1-{3-[4-(5-Méthoxypyrimidin-4-yl)pipérazin-1-yl]propyl}-7-([1,2,4] triazol-4-yl)indazole, dichlorhydrate**

***ETUDE PHARMACOLOGIQUE DES COMPOSES DE L'INVENTION***

***EXEMPLE A* : Contraction de la veine saphène**

Les expériences sont effectuées sur des veines saphènes de chiens (10-25 kg) ou de lapins (2-3 kg) anesthésiés avec du pentobarbital (30 mg/kg i.v.). Les veines saphènes sont rapidement prélevées et découpées en anneaux. Ces anneaux sont montés entre deux crochets dans des cuves thermostatées à 37°C contenant de la solution physiologique (composition en mM : NaCl 118.3 ; KCl 4.7 ; $CaCl_2$ 2.5 ; $MgSO_4$ 1.2 ; $KH_2PO_4$ 1.2 ; Na $HCO_3$ 25.0 ; Ca-EDTA 0.026 et glucose 11.1).

La solution physiologique est bullée par un mélange de 95 % $O_2$ - 5 % $CO_2$. Le crochet inférieur consiste le point fixe tandis que le crochet supérieur est relié à un capteur de force isométrique. Les tissus sont mis sous une tension de base de 1.5 grammes (chien) et de 1 gramme (lapin). Les substances pharmacologiques étudiées sont préparées immédiatement avant l'usage ; elles sont solubilisées dans l'eau ou dans le diméthyl sulfoxyde. Après le montage, les préparations sont laissées en repos pendant 60 minutes, des rinçages étant effectués toutes les 30 minutes. L'organe est alors mis en présence de phénoxybenzamine ($5 \times 10^{-8}$M) pendant 20 minutes. Cet agent est éliminé par plusieurs lavages successifs pendant 45 minutes. Après réajustement de la tension de base, une contraction est provoquée par le KCl (100 mM). Après lavage et retour à la ligne de base, une contraction est induite par la 5-hydroxytryptamine ($10^{-5}$M).

Après lavage et retour à la ligne de base, une courbe dose-réponse aux substances pharmacologiques est effectuée par adjonction de doses cumulatives ($10^{-9}$ à $10^{-4}$M).

Cette expérience permet de calculer la concentration efficace 50 % ($EC_{50}$) des composés de l'invention.

Cette $EC_{50}$ est calculée de la manière suivante : les valeurs de tension sont d'abord converties en pourcentages par rapport à l'effet maximum induit par le KCl. La concentration efficace 50 % ($EC_{50}$) est déterminée par régression non linéaire suivant le modèle de la loi d'action de masse de Michaelis-Menten.

Résultats

Les composés des exemples 5 et 6 contractent la veine saphène de lapin avec des $EC_{50}$ de 0,30 µM et 0,12 µM respectivement et des réponses maximales de 75 % et 87 % respectivement.
Sur la veine saphène de chien, 1' $EC_{50}$ du composé de l'exemple 6 est de 0,17 µM et la réponse maximale de 47 %. Le composé de l'exemple 5 provoque une contraction maximale de 6 % à une concentration de 100 µM.

***EXEMPLE B** : Compositions pharmaceutiques*

Formule de préparation pour 1000 comprimés dosés à 10 mg :

| | |
|---|---|
| Composé de l'exemple 5 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

**1.** Composés de formule (I):

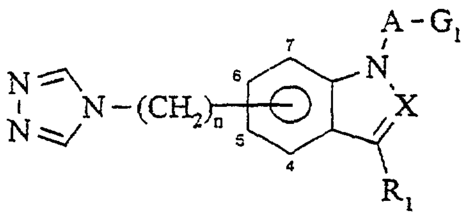

(I)

dans laquelle :

- n est égal à 0 ou 1,
- A représente une liaison σ ou un groupement alkylène ($C_1$-$C_8$) linéaire ou ramifié, ou un groupement alkénylène ($C_2$-$C_8$) linéaire ou ramifié,
- X représente un atome d'azote ou un groupement C-$R_2$ dans lequel $R_2$ représente un atome d'hydrogène, ou un groupement alkyle ($C_1$-$C_6$) linéaire ou ramifié,
- $R_1$ représente un atome d'hydrogène ou un groupement alkyle ($C_1$-$C_6$) linéaire ou ramifié,
- $G_1$ représente un groupement pyrrolidinyle ou pipéridyle, chacun de ces groupements étant éventuellement substitué sur l'un quelconque des sommets du cycle par un groupement alkyle ($C_1$-$C_6$) linéaire ou ramifié, cycloalkyle ($C_3$-$C_7$), cycloalkyle ($C_3$-$C_7$) alkyle ($C_1$-$C_6$) linéaire ou ramifié, nitrile, carboxy, alkoxy ($C_1$-$C_6$) carbonyle linéaire ou ramifié, carbamoyle (éventuellement substitué par un ou deux groupements alkyle ($C_1$-$C_6$) linéaire ou ramifié, cycloalkyle ($C_3$-$C_7$), phényle éventuellement substitué, et/ou benzyle éventuellement substitué), aryle éventuellement substitué, arylalkyle ($C_1$-$C_6$) linéaire ou ramifié éventuellement substitué, hétéroaryle éventuellement substitué, ou hétéroarylalkyle ($C_1$-$C_6$) linéaire ou ramifié éventuellement substitué,

étant entendu que ces groupements pyrrolidine et pipéridine peuvent être liés à A par l'un quelconque des atomes du cycle,
ou bien, $G_1$ représente un groupement

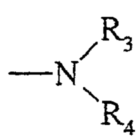

dans lequel :

$R_3$ et $R_4$ représentent indépendamment un atome d'hydrogène, ou un groupement alkyle ($C_1$-$C_6$) linéaire ou ramifié, cycloalkyle ($C_3$-$C_7$), cycloalkyl ($C_3$-$C_7$) alkyle ($C_1$-$C_6$) linéaire ou ramifié, arylalkyle ($C_1$-$C_6$) linéaire ou ramifié éventuellement substitué, aryle éventuellement substitué, ou forment ensemble avec l'atome d'azote

qui les porte un groupement :

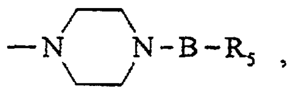

pour lequel B représente une liaison σ ou un groupement alkylène ($C_1$-$C_6$) linéaire ou ramifié, et $R_5$ représente un atome d'hydrogène, ou un groupement alkyle ($C_1$-$C_6$) linéaire ou ramifié, cycloalkyle ($C_3$-$C_7$), aryle éventuellement substitué, arylalkyle ($C_1$-$C_6$) linéaire ou ramifié éventuellement substitué, hétéroaryle éventuellement substitué, ou hétéroarylalkyle ($C_1$-$C_6$) linéaire ou ramifié éventuellement substitué,

leurs énantiomères, diastéréoisomères, ainsi que leurs sels d'addition à un acide ou à une base pharmaceutiquement acceptable,

étant entendu que :

- par groupement aryle, on entend un groupement choisi parmi phényle et naphtyle,
- par groupement hétéroaryle, on entend un groupement choisi parmi furyle, thiényle, pyridyle, pyrrolyle, imidazolyle, tétrazolyle, pyrazinyle et pyrimidinyle,
- et que le terme *éventuellement substitué* affectant les expressions phényle, benzyle, aryle, arylalkyle, hétéroaryle, ou hétéroarylalkyle signifie que les groupements concernés sont substitués par un ou plusieurs atomes d'halogène, et/ou groupement alkyle ($C_1$-$C_6$) linéaire ou ramifié, alkoxy ($C_1$-$C_6$) linéaire ou ramifié, hydroxy, et/ou trihalogénoalkyle ($C_1$-$C_6$) linéaire ou ramifié.

**2.** Composés de formule (I) selon la revendication 1 tels que le groupement triazolyle ou triazolylméthyle est relié en position 6 du noyau indole ou indazole, leurs énantiomères, diastéréoisomères, ainsi que leurs sels d'addition à un acide ou à une base pharmaceutiquement acceptable.

**3.** Composés de formule (I) selon la revendication 1 tels que le groupement triazolyle ou triazolylméthyle est relié en position 5 du noyau indole ou indazole, leurs énantiomères, diastéréoisomères, ainsi que leurs sels d'addition à un acide ou à une base pharmaceutiquement acceptable.

**4.** Composés de formule (I) selon la revendication 1 tels que n = 0, leurs énantiomères, diastéréoisomères, ainsi que leurs sels d'addition à un acide ou à une base pharmaceutiquement acceptable.

**5.** Composés de formule (I) selon la revendication 1 tels que X représente un groupement C-$R_2$, dans lequel $R_2$ représente un atome d'hydrogène, ou un groupement alkyle ($C_1$-$C_6$) linéaire ou ramifié, leurs énantiomères, diastéréoisomères, ainsi que leurs sels d'addition à un acide ou à une base pharmaceutiquement acceptable.

**6.** Composés de formule (I) selon la revendication 1 tels que X représente un atome d'azote, leurs énantiomères, diastéréoisomères, ainsi que leurs sels d'addition à un acide ou à une base pharmaceutiquement acceptable.

**7.** Composés de formule (I) selon la revendication 1 pour lesquels A représente une liaison σ ou un groupement alkylène ($C_1$-$C_6$) linéaire ou ramifié, et $G_1$ représente un groupement pyrrolidinyle ou pipéridinyle, chacun de ces groupements étant éventuellement substitués sur l'un quelconque des sommets du cycle par un groupement alkyle ($C_1$-$C_6$) linéaire ou ramifié, cycloalkyle ($C_3$-$C_7$), cycloalkyle ($C_3$-$C_7$) alkyle ($C_1$-$C_6$) linéaire ou ramifié, nitrile, carboxy, alkoxy($C_1$-$C_6$)carbonyle linéaire ou ramifié, carbamoyle (éventuellement substitué par un ou deux groupements alkyle ($C_1$-$C_6$) linéaire ou ramifié, cycloalkyle ($C_3$-$C_7$), phényle éventuellement substitué, et/ou benzyle éventuellement substitué), aryle éventuellement substitué, arylalkyle ($C_1$-$C_6$) linéaire ou ramifié éventuellement substitué, hétéroaryle éventuellement substitué, ou hétéroarylalkyle ($C_1$-$C_6$) linéaire ou ramifié éventuellement substitué,
ou bien $G_1$ représente un groupement

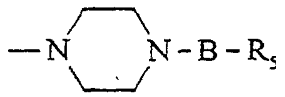

, pour lequel B représente une liaison σ ou un groupement alkylène ($C_1$-$C_6$) linéaire ou ramifié, et $R_5$ représente un atome d'hydrogène, ou un groupement alkyle ($C_1$-$C_6$) linéaire ou ramifié, cycloalkyle ($C_3$-$C_7$), aryle éventuellement substitué, arylalkyle ($C_1$-$C_6$) linéaire ou ramifié éventuellement substitué, hétéroaryle éventuellement substitué, ou hétéroarylalkyle ($C_1$-$C_6$) linéaire ou ramifié éventuellement substitué, leurs énantiomères, diastéréoisomères, ainsi que leurs sels d'addition à un acide ou à une base pharmaceutiquement acceptable.

**8.** Composé de formule (I) selon la revendication 1 pour lesquels A représente un groupement alkylène ($C_1$-$C_6$) linéaire ou ramifié et $G_1$ représente un groupement

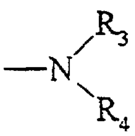

dans lequel $R_3$ et $R_4$ représentent indépendamment un atome d'hydrogène, ou un groupement alkyle ($C_1$-$C_6$) linéaire ou ramifié, cycloalkyle ($C_3$-$C_7$), cycloalkyl ($C_3$-$C_7$) alkyle ($C_1$-$C_6$) linéaire ou ramifié, arylalkyle ($C_1$-$C_6$) linéaire ou ramifié éventuellement substitué, aryle éventuellement substitué, leurs énantiomères, diastéréoisomères, ainsi que leurs sels d'addition à un acide ou à une base pharmaceutiquement acceptable.

**9.** Composés de formule (I) selon la revendication 1 pour lesquels le groupement triazolyle ou triazolylméthyle est rattaché en position 6 du noyau indole ou indazole, X représente un groupement -CH- ou un atome d'azote, R, représente un atome d'hydrogène, A représente une liaison σ ou un groupement alkylène ($C_1$-$C_6$) linéaire ou ramifié tandis que $G_1$ représente un groupement pyrrolidinyle ou pipéridinyle éventuellement substitués sur l'un quelconque des sommets du cycle par un groupement alkyle ($C_1$-$C_6$) linéaire ou ramifié, aryle éventuellement substitué, arylalkyle ($C_1$-$C_6$) linéaire ou ramifié éventuellement substitué, hétéroaryle éventuellement substitué, ou hétéroarylalkyle ($C_1$-$C_6$) linéaire ou ramifié éventuellement substitué, ou $G_1$ représente un groupement 1-pipérazinyle éventuellement substitué sur l'atome d'azote en position 4 par un groupement alkyle ($C_1$-$C_6$) linéaire ou ramifié, aryle éventuellement substitué, arylalkyle ($C_1$-$C_6$) linéaire ou ramifié éventuellement substitué, hétéroaryle éventuellement substitué, ou hétéroarylalkyle ($C_1$-$C_6$) linéaire ou ramifié éventuellement substitué,
ou bien A représente un groupement alkylène ($C_1$-$C_6$) linéaire ou ramifié tandis que $G_1$ représente un groupement

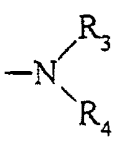

dans lequel $R_3$ et $R_4$ représentent indépendamment un atome d'hydrogène, ou un groupement alkyle ($C_1$-$C_6$) linéaire ou ramifié, leurs énantiomères, diastéréoisomères, ainsi que leurs sels d'addition à un acide ou à une base pharmaceutiquement acceptable.

**10.** Composé de formule (I) selon la revendication 1 qui est le 1-(diméthylaminoéthyl)-6-([1,2,4]triazol-4-yl)indole, ainsi que ses sels d'addition à un acide pharmaceutiquement acceptable.

**11.** Composé de formule (I) selon la revendication 1 qui est le 1-(diméthylaminoéthyl)-6-([1,2,4]triazol-4-yl)indazole, ainsi que ses sels d'addtion à un acide pharmaceutiquement acceptable.

**12.** Composé de formule (I) selon la revendication 1 qui est le 1-{3-[4-(5-méthoxypyrimidin-4-yl)pipérazin-1-yl]propyl}-6-([1,2,4]triazol-4-yl)indole, ainsi que ses sels d'addtion à un acide pharmaceutiquement acceptable.

**13.** Composé de formule (I) selon la revendication 1 qui est le 1-{3-[4-(5-méthoxypyrimidin-4-yl)pipérazin-1-yl]propyl}-6-([1,2,4]triazol-4-yl)indazole, ainsi que ses sels d'addtion à un acide pharmaceutiquement acceptable.

**14.** Procédé de préparation des composés de formule (I) caractérisé,

♦ lorsque dans le composé de formule (I) que l'on souhaite obtenir, X représente un groupement C-$R_2$, en ce que l'on utilise comme produit de départ: un composé de formule (II/a):

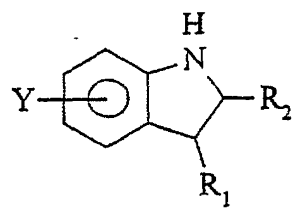

(II/a)

dans laquelle $R_1$ et $R_2$ sont tels que définis dans la formule (I) et Y représente un groupement nitro si dans le composé de formule (I) que l'on souhaite obtenir n vaut 0, ou un groupement hydroxyméthyle si dans le composé de formule (I) que l'on souhaite obtenir, n vaut 1,
qui est condensé,

- ***soit*** sur un dérivé de formule (III/a):

$$Z - A - G_1 \quad \text{(III/a)}$$

dans laquelle A et $G_1$ sont tels que définis dans la formule (I) et Z représente un atome d'halogène ou un groupement formyle,
pour conduire à un composé de formule (IV) :

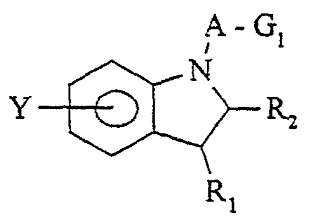

(IV)

dans laquelle A, Y, $G_1$, $R_1$, $R_2$ sont tels que définis précédemment, qui subit une réaction d'oxydation pour conduire à un composé de formule (V/a) :

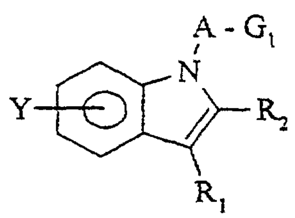

(V/a.)

dans laquelle A, Y, $G_1$, $R_1$, $R_2$ sont tels que définis précédemment, dont le groupement $G_1$, lorsqu'il représente un groupement pyrrolidinyle, pipéridyle ou pipérazinyle, peut être substitué sur l'atome d'azote, en milieu basique ou par une réaction d'amination réductrice sur un dérivé carbonylé,

- ***soit*** sur un dérivé de formule (III/b), en milieu basique :

Br-A-OH (III/b)

dans laquelle A a la même signification que précédemment, pour conduire à un composé de formule (IV') :

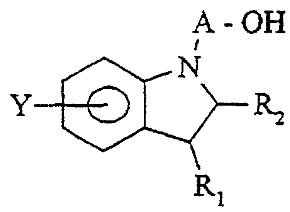
(IV')

dans laquelle Y, A, $R_1$, $R_2$ sont tels que définis précédemment, qui, après bromation et oxydation, est condensé sur un dérivé de formule $G_1H$, $G_1$ ayant la même signification que dans la formule (I), pour conduire à un composé de formule (V/a), telle que définie précédemment,

ou bien,

♦ lorsque dans le composé de formule (I) que l'on souhaite obtenir, X représente un atome d'azote, en ce que l'on utilise comme matière première un composé de formule (II/b):

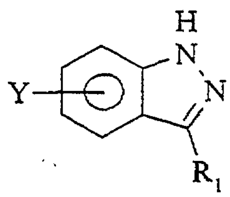
(II/b)

dans laquelle Y et $R_1$ sont tels que définis précédemment,
qui est condensé,

- *soit* sur un dérivé de formule (III/c), en milieu basique :

Hal - A - $G_1$ (III/c)

dans laquelle A et $G_1$ sont tels que définis précédemment, et Hal représente un atome d'halogène, pour conduire à un composé de formule (V/b):

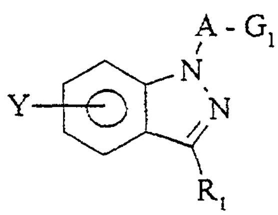
(V/b)

dans laquelle A, Y, $G_1$ et $R_1$ sont tels que définis précédemment, dont le groupement $G_1$, lorsqu'il représente un groupement pyrrolidinyle, pipéridyle ou pipérazinyle, peut être substitué sur l'atome d'azote, en milieu basique ou par une réaction d'amination réductrice sur un dérivé carbonylé,

- ***soit*** sur un dérivé de formule (III/b) telle que définie précédemment, pour conduire à un composé de

formule (VI) :

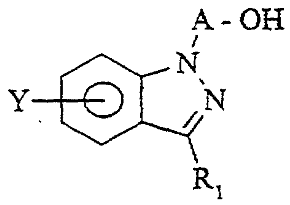

(VI)

dans laquelle Y, A et $R_1$ sont tels que définis précédemment, qui, après bromation, est condensé sur un dérivé de formule $HG_1$, $G_1$ ayant la même signification que dans la formule (I), pour conduire à un composé de formule (V/b) telle que définie précédemment,
composés de formule (V/a) et (V/b) formant la totalité des composés de formule (V):

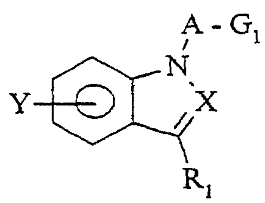

(V)

dans laquelle X, Y, A, $R_1$, et $G_1$ sont tels que définis dans la formule (I), et Y est tel que défini précédemment,
qui, par réduction du groupement Y (lorsque Y représente un groupement hydroxyméthyle, après passage par un intermédiaire de type azido), conduit à un composé de formule (VII):

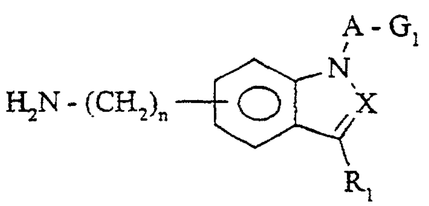

(VII)

dans laquelle n, X, A, $G_1$ et $R_1$ sont tels que définis précédemment,
qui est condensé sur la N,N-diméthylformamide azine, en milieu acide, pour conduire aux composés de formule (I),

- que l'on purifie éventuellement selon une technique classique de purification,
- dont on sépare éventuellement les énantiomères selon une technique classique de séparation,
- et que l'on transforme, le cas échéant, en leurs sels d'addition à un acide ou à une base pharmaceutiquement acceptable,
- étant entendu que lorsque Y contient un groupement hydroxy, ce dernier peut être protégé et déprotégé en fonction des réactifs utilisés.

**15.** Compositions pharmaceutiques contenant comme principe actif au moins un composé selon l'une quelconque des revendications 1 à 13, seul ou en combinaison avec un ou plusieurs excipients ou véhicules inertes, non toxiques, pharmaceutiquement acceptables.

**16.** Compositions pharmaceutiques selon la revendication 15 contenant au moins un principe actif selon l'une quelconque des revendications 1 à 13 utiles dans le traitement de l'insuffisance veineuse et des maladies associées et/ou dans le traitement de la migraine et de la migraine associée aux maladies vasculaires.

**Claims**

1. Compounds of formula (I) :

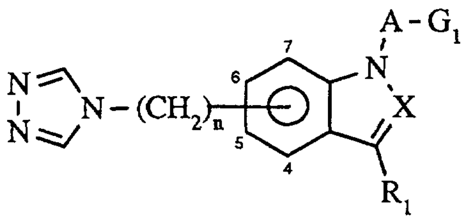

(I)

wherein:

- n is 0 or 1,
- A represents a σ bond or a linear or branched ($C_1$-$C_8$)alkylene group or a linear or branched ($C_2$-$C_8$)alkenylene group,
- X represents a nitrogen atom or a C-$R_2$ group wherein $R_2$ represents a hydrogen atom or a linear or branched ($C_1$-$C_6$)alkyl group,
- $R_1$ represents a hydrogen atom or a linear or branched ($C_1$-$C_6$)alkyl group,
- $G_1$ represents a pyrrolidinyl or piperidyl group, each of those groups being optionally substituted on any of the junctions of the ring by a linear or branched ($C_1$-$C_6$)alkyl group, ($C_3$-$C_7$)cycloalkyl group, ($C_3$-$C_7$)cycloalkyl-($C_1$-$C_6$)alkyl group in which the alkyl is linear or branched, nitrile group, carboxy group, linear or branched ($C_1$-$C_6$)alkoxycarbonyl group, carbamoyl group (optionally substituted by one or two linear or branched ($C_1$-$C_6$)alkyl, ($C_3$-$C_7$)cycloalkyl, optionally substituted phenyl and/or optionally substituted benzyl groups), optionally substituted aryl group, optionally substituted aryl-($C_1$-$C_6$)alkyl group in which the alkyl is linear or branched, optionally substituted heteroaryl group or optionally substituted heteroaryl-($C_1$-$C_6$)alkyl group in which the alkyl is linear or branched,

it being understood that those pyrrolidine and piperidine groups may be bonded to A by any one of the atoms of the ring,
or $G_1$ represents a group

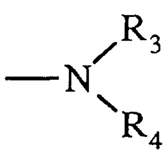

group wherein:

$R_3$ and $R_4$ each independently of the other represent a hydrogen atom, or a linear or branched ($C_1$-$C_6$)alkyl group, ($C_3$-$C_7$)cycloalkyl group, ($C_3$-$C_7$)cycloalkyl-($C_1$-$C_6$)-alkyl group in which the alkyl is linear or branched, optionally substituted aryl-($C_1$-$C_6$)alkyl group in which the alkyl is linear or branched or optionally substituted aryl group, or, together with the nitrogen atom that carries them, form a group :

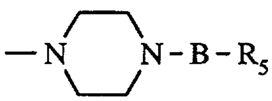

,

wherein B represents a σ bond or a linear or branched ($C_1$-$C_6$)alkylene group, and $R_5$ represents a hydrogen atom, or a linear or branched ($C_1$-$C_6$)alkyl group, ($C_3$-$C_7$)cycloalkyl group, optionally substituted aryl group, optionally substituted aryl-($C_1$-$C_6$)alkyl group in which the alkyl is linear or branched, optionally substituted heteroaryl group or optionally substituted heteroaryl-($C_1$-$C_6$)alkyl group in which the alkyl is linear or

branched,

their enantiomers and diastereoisomers, and addition salts thereof with a pharmaceutically acceptable acid or base,

it being understood that:

- "aryl group" is understood to mean a group selected from phenyl and naphthyl,
- "heteroaryl group" is understood to mean a group selected from furyl, thienyl, pyridyl, pyrrolyl, imidazolyl, tetrazolyl, pyrazinyl and pyrimidinyl,
- and the term *optionally substituted* applied to the terms "phenyl", "benzyl", "aryl", "arylalkyl", "heteroaryl" and "heteroarylalkyl" means that the groups in question are substituted by one or more halogen atoms and/or linear or branched ($C_1$-$C_6$)alkyl, linear or branched ($C_1$-$C_6$)alkoxy, hydroxy and/or linear or branched ($C_1$-$C_6$)-trihaloalkyl groups.

**2.** Compounds of formula (I) according to claim 1 wherein the triazolyl or triazolylmethyl group is attached to the 6-position of the indole or indazole group, their enantiomers and diastereoisomers, and addition salts thereof with a pharmaceutically acceptable acid or base.

**3.** Compounds of formula (I) according to claim 1 wherein the triazolyl or triazolylmethyl group is attached to the 5-position of the indole or indazole group, their enantiomers and diastereoisomers, and addition salts thereof with a pharmaceutically acceptable acid or base.

**4.** Compounds of formula (I) according to claim 1 wherein n = 0, their enantiomers and diastereoisomers, and addition salts thereof with a pharmaceutically acceptable acid or base.

**5.** Compounds of formula (I) according to claim 1 wherein X represents a C-$R_2$ group wherein $R_2$ represents a hydrogen atom or a linear or branched ($C_1$-$C_6$)alkyl group, their enantiomers and diastereoisomers, and addition salts thereof with a pharmaceutically acceptable acid or base.

**6.** Compounds of formula (I) according to claim 1 wherein X represents a nitrogen atom, their enantiomers and diastereoisomers, and addition salts thereof with a pharmaceutically acceptable acid or base.

**7.** Compounds of formula (I) according to claim 1 wherein A represents a σ bond or a linear or branched ($C_1$-$C_6$) alkylene group, and $G_1$ represents a pyrrolidinyl or piperidyl group, each of those groups being optionally substituted on any of the junctions of the ring by a linear or branched ($C_1$-$C_6$)alkyl group, ($C_3$-$C_7$)cycloalkyl group, ($C_3$-$C_7$)cycloalkyl-($C_1$-$C_6$)alkyl group in which the alkyl is linear or branched, nitrile group, carboxy group, linear or branched ($C_1$-$C_6$)alkoxycarbonyl group, carbamoyl group (optionally substituted by one or two linear or branched ($C_1$-$C_6$)-alkyl, ($C_3$-$C_7$)cycloalkyl, optionally substituted phenyl and/or optionally substituted benzyl groups), optionally substituted aryl group, optionally substituted aryl-($C_1$-$C_6$)-alkyl group in which the alkyl is linear or branched, optionally substituted heteroaryl group or optionally substituted heteroaryl-($C_1$-$C_6$)alkyl group in which the alkyl is linear or branched,
or $G_1$ represents a group

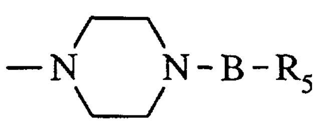

, wherein B represents a σ bond or a linear or branched ($C_1$-$C_6$)alkylene group, and $R_5$ represents a hydrogen atom or a linear or branched ($C_1$-$C_6$)alkyl group, ($C_3$-$C_7$)cycloalkyl group, optionally substituted aryl group, optionally substituted aryl-($C_1$-$C_6$)alkyl group in which the alkyl is linear or branched, optionally substituted heteroaryl group or optionally substituted heteroaryl-($C_1$-$C_6$)alkyl group in which the alkyl is linear or branched, their enantiomers and diastereoisomers, and addition salts thereof with a pharmaceutically acceptable acid or base.

**8.** Compound of formula (I) according to claim 1 wherein A represents a linear or branched ($C_1$-$C_6$)alkylene group and $G_1$ represents a group

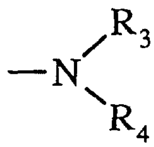

wherein $R_3$ and $R_4$ each independently of the other represent a hydrogen atom or a linear or branched ($C_1$-$C_6$) alkyl group, ($C_3$-$C_7$)cycloalkyl group, ($C_3$-$C_7$)cycloalkyl-($C_1$-$C_6$)alkyl group in which the alkyl is linear or branched, optionally substituted aryl-($C_1$-$C_6$)alkyl group in which the alkyl is linear or branched or optionally substituted aryl group, their enantiomers and diastereoisomers, and addition salts thereof with a pharmaceutically acceptable acid or base.

**9.** Compounds of formula (I) according to claim 1 wherein the triazolyl or triazolylmethyl group is attached to the 6-position of the indole or indazole group, X represents a -CH- group or a nitrogen atom, $R_1$ represents a hydrogen atom, A represents a σ bond or a linear or branched ($C_1$-$C_6$)alkylene group whilst $G_1$ represents a pyrrolidinyl or piperidyl group optionally substituted on any of the junctions of the ring by a linear or branched ($C_1$-$C_6$)alkyl group, optionally substituted aryl group, optionally substituted aryl-($C_1$-$C_6$)alkyl group in which the alkyl is linear or branched, optionally substituted heteroaryl group or optionally substituted heteroaryl-($C_1$-$C_6$)alkyl group in which the alkyl is linear or branched, or $G_1$ represents a 1-piperazinyl group optionally substituted on the nitrogen atom in the 4-position by a linear or branched ($C_1$-$C_6$)alkyl group, optionally substituted aryl group, optionally substituted aryl-($C_1$-$C_6$)alkyl group in which the alkyl is linear or branched, optionally substituted heteroaryl group or optionally substituted heteroaryl-($C_1$-$C_6$)-alkyl group in which the alkyl is linear or branched, or A represents a linear or branched ($C_1$-$C_6$)alkylene group whilst $G_1$ represents a group

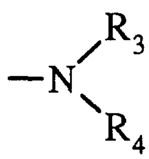

wherein $R_3$ and $R_4$ each independently of the other represent a hydrogen atom or a linear or branched ($C_1$-$C_6$) alkyl group, their enantiomers and diastereoisomers, and addition salts thereof with a pharmaceutically acceptable acid or base.

**10.** Compound of formula (I) according to claim 1 which is 1-(dimethylaminoethyl)-6-([1,2,4]triazol-4-yl)indole, and its addition salts with a pharmaceutically acceptable acid.

**11.** Compound of formula (I) according to claim 1 which is 1-(dimethylaminoethyl)-6-([1,2,4]triazol-4-yl)indazole, and its addition salts with a pharmaceutically acceptable acid.

**12.** Compound of formula (I) according to claim 1 which is 1-{3-[4-(5-methoxypyrimidin-4-yl)piperazin-1-yl]propyl}-6-([1,2,4]triazol-4-yl)indole, and its addition salts with a pharmaceutically acceptable acid.

**13.** Compound of formula (I) according to claim 1 which is 1-{3-[4-(5-methoxypyrimidin-4-yl)piperazin-1-yl]propyl}-6-([1,2,4]triazol-4-yl)indazole, and its addition salts with a pharmaceutically acceptable acid.

**14.** Process for the preparation of compounds of formula (I) characterised in that,

- ♦ when, in the desired compound of formula (I), X represents a C-$R_2$ group, there is used as starting material a compound of formula (II/a) :

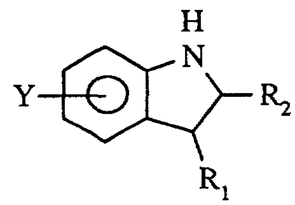

(II/a)

wherein $R_1$ and $R_2$ are as defined for formula (I) and Y represents a nitro group if, in the desired compound of formula (I), n is 0, or Y represents a hydroxymethyl group if, in the desired compound of formula (I), n is 1, which is condensed,

- ***either*** with a compound of formula (III/a):

$$Z - A - G_1 \qquad \text{(III/a)}$$

wherein A and $G_1$ are as defined for formula (I) and Z represents a halogen atom or a formyl group, to yield a compound of formula (IV) :

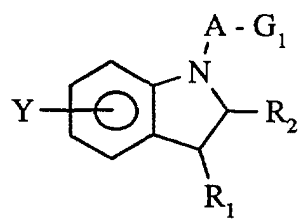

(IV)

wherein A, Y, $G_1$, $R_1$ and $R_2$ are as defined hereinabove, which is subjected to an oxidation reaction to yield a compound of formula (V/a):

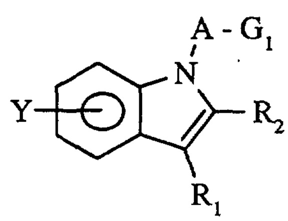

(V/a)

wherein A, Y, $G_1$, $R_1$ and $R_2$ are as defined hereinabove, and the $G_1$ group, when it represents a pyrrolidinyl, piperidyl or piperazinyl group, may be substituted on the nitrogen atom in a basic medium or by a reductive amination reaction with a carbonyl compound,

- ***or*** with a compound of formula (III/b), in a basic medium :

$$Br - A - OH \qquad \text{(III/b)}$$

wherein A is as defined hereinabove, to yield a compound of formula (IV') :

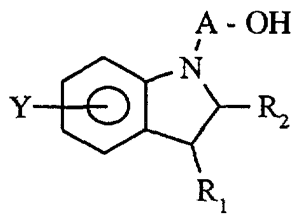

(IV')

wherein Y, A, $R_1$ and $R_2$ are as defined hereinabove, which, after bromination and oxidation, is condensed with a compound of formula $G_1H$, $G_1$ being as defined for formula (I), to yield a compound of formula (V/a), as defined hereinabove,

or,

♦ when, in the desired compound of formula (I), X represents a nitrogen atom, there is used as starting material a compound of formula (II/b) :

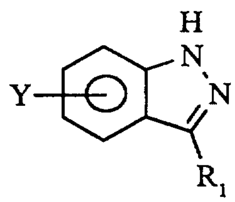

(II/b)

wherein Y and $R_1$ are as defined hereinabove, which is condensed

- ***either*** with a compound of formula (III/c), in a basic medium :

$$Hal - A - G_1 \qquad (III/c)$$

wherein A and $G_1$ are as defined hereinabove, and Hal represents a halogen atom, to yield a compound of formula (V/b):

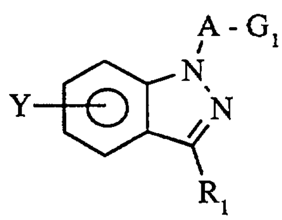

(V/b)

wherein A, Y, $G_1$ and $R_1$ are as defined hereinabove, and the $G_1$ group, when it represents a pyrrolidinyl, piperidyl or piperazinyl group, may be substituted on the nitrogen atom, in a basic medium or by a reductive amination reaction with a carbonyl compound,

- ***or*** with a compound of formula (III/b) as defined hereinabove, to yield a compound of formula (VI) :

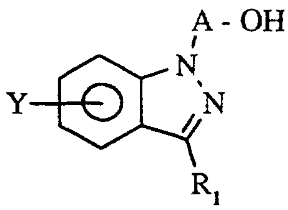

(VI)

wherein Y, A and $R_1$ are as defined hereinabove, which, after bromination, is condensed with a compound of formula $HG_1$, $G_1$ being as defined for formula (I), to yield a compound of formula (V/b) as defined hereinabove, which compounds of formulae (V/a) and (V/b) constitute the totality of the compounds of formula (V):

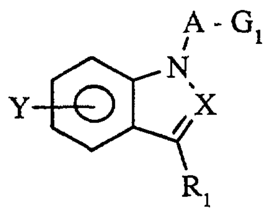

(V)

wherein X, Y, A, $R_1$ and $G_1$ are as defined for formula (I) and Y is as defined hereinabove,
which, after reduction of the Y group (when Y represents a hydroxymethyl group, *via* an azide-type intermediate), yields a compound of formula (VII) :

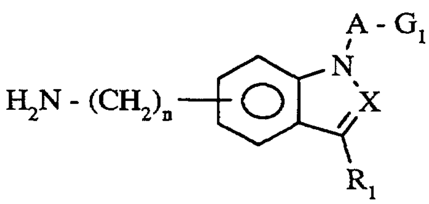

(VII)

wherein n, X, A, $G_1$ and $R_1$ are as defined hereinabove,
which is condensed with N,N-dimethylformamide azine, in an acidic medium, to yield the compounds of formula (I),

- which are optionally purified in accordance with a conventional purification technique,
- optionally separated into their enantiomers in accordance with a conventional separation technique, and
- converted, where appropriate, into addition salts thereof with a pharmaceutically acceptable acid or base,
- it being understood that when Y contains a hydroxy group, the latter may be protected or deprotected depending on the reagents used.

**15.** Pharmaceutical compositions comprising as active ingredient at least one compound according to any one of claims 1 to 13, alone or in combination with one or more inert, non-toxic, pharmaceutically acceptable excipients or carriers.

**16.** Pharmaceutical compositions according to claim 15 comprising at least one active ingredient according to any one of claims 1 to 13 for use in the treatment of venous insufficiency and of associated disorders and/or in the treatment of migraine and of migraine associated with vascular diseases.

## Patentansprüche

**1.** Verbindungen der Formel (I):

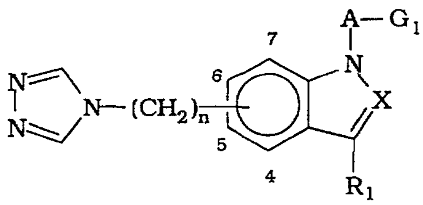

(I)

in der:

- n 0 oder 1 bedeutet,
- A eine σ-Bindung oder eine geradkettige oder verzweigte ($C_1$-$C_8$)-Alkylengruppe oder eine geradkettige oder verzweigte ($C_2$-$C_8$)-Alkenylengruppe darstellt,
- ein Stickstoffatom oder eine Gruppe C-$R_2$ darstellt, worin $R_2$ ein Wasserstoffatom oder eine geradkettige oder verzweigte ($C_1$-$C_6$)-Alkylgruppe bedeutet,
- $R_1$ ein Wasserstoffatom oder eine geradkettige oder verzweigte ($C_1$-$C_6$)-Alkyl-gruppe darstellt,
- $G_1$ eine Pyrrolidinyl- oder Piperidylgruppe darstellt, wobei jede dieser Gruppen gegebenenfalls an einem der Ringatome substituiert sein kann durch eine geradkettige oder verzweigte ($C_1$-$C_6$)-Alkylgruppe, ($C_3$-$C_7$)-Cycloalkylgruppe, geradkettige oder verzweigte ($C_1$-$C_6$)-Alkyl-($C_3$-$C_7$)-cycloalkylgruppe, Nitrilgruppe, Carboxygruppe, geradkettige oder verzweigte ($C_1$-$C_6$)-Alkoxycarbonylgruppe, Carbamoylgruppe (die gegebenenfalls durch eine oder zwei geradkettige oder verzweigte ($C_1$-$C_6$)-Alkylgruppen, ($C_3$-$C_7$)-Cycloalkylgruppen, gegebenenfalls substituierte Phenylgruppen und/oder gegebenenfalls substituierte Benzylgruppen substituiert sein kann), gegebenenfalls substituierte Arylgruppe, gegebenenfalls substituierte geradkettige oder verzweigte Aryl-($C_1$-$C_6$)-alkylgruppe, gegebenenfalls substituierte Heteroarylgruppe oder gegebenenfalls substituierte geradkettige oder verzweigte Heteroaryl-($C_1$-$C_6$)-alkylgruppe substituiert ist, mit der Maßgabe, daß die Pyrrolidin- und Piperidingruppen über irgendeines der Ringatome an A gebunden sein können,
  oder $G_1$ eine Gruppe

  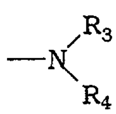

  darstellt, in der:

  $R_3$ und $R_4$ unabhängig voneinander Wasserstoffatome oder geradkettige oder verzweigte ($C_1$-$C_6$)-Alkylgruppen, ($C_3$-$C_7$)-Cycloalkylgruppen, geradkettige oder verzweigte ($C_3$-$C_7$)-Cycloalkyl-($C_1$-$C_6$)-alkylgruppen, geradkettige oder verzweigte und gegebenenfalls substituierte Aryl-($C_1$-$C_6$)-alkylgruppen oder gegebenenfalls substituierte Arylgruppen bedeuten oder gemeinsam mit dem sie tragenden Stickstoffatom eine Gruppe:

  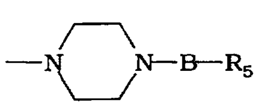

  bilden, in der B eine σ-Bindung oder eine geradkettige oder verzweigte ($C_1$-$C_6$)-Alkylengruppe und $R_5$ ein Wasserstoffatom oder eine geradkettige oder verzweigte ($C_1$-$C_6$)-Alkylgruppe, ($C_3$-$C_7$)-Cycloalkylgruppe, gegebenenfalls substituierte Arylgruppe, geradkettige oder verzweigte und gegebenenfalls substituierte Aryl-($C_1$-$C_6$)-alkylgruppe, gegebenenfalls substituierte Heteroarylgruppe oder geradkettige oder verzweigte und gegebenenfalls substituierte Heteroaryl-($C_1$-$C_6$)-alkylgruppe bedeuten,
  deren Enantiomere, Diastereoisomere sowie deren Additionssalze mit einer pharmazeutisch annehmbaren Säure oder Base, mit der Maßgabe, daß:

- unter einer Arylgruppe eine Gruppe ausgewählt aus Phenyl und Naphthyl zu verstehen ist,
- unter einer Heteroarylgruppe eine Gruppe ausgewählt aus Furyl, Thienyl, Pyridyl, Pyrrolyl, Imidazolyl, Tetrazolyl, Pyrazinyl und Pyrimidinyl zu verstehen ist,
- und der Begriff *gegebenenfalls substituiert* bezüglich der Ausdrücke Phenyl, Benzyl, Aryl, Arylalkyl, Heteroaryl oder Heteroarylalkyl bedeutet, daß die betreffenden Gruppen durch ein oder mehrere Halogenatome und/oder geradkettige oder verzweigte ($C_1$-$C_6$)-Alkylgruppen, geradkettige oder verzweigte ($C_1$-$C_6$)-Alkoxygruppen, Hydroxygruppen und/oder geradkettige oder verzweigte ($C_1$-$C_6$)-Trihalogenalkylgruppen substituiert sind.

**2.** Verbindungen der Formel (I) nach Anspruch 1, bei denen die Triazolylgruppe oder die Triazolylmethylgruppe in der 6-Stellung des Indol- oder Indazolkerns gebunden ist, deren Enantiomere, Diastereoisomere sowie deren Additionssalze mit einer pharmazeutisch annehmbaren Säure oder Base.

**3.** Verbindungen der Formel (I) nach Anspruch 1, worin die Triazolylgruppe oder die Triazolylmethylgruppe in der 5-Stellung des Indol- oder Indazolkerns gebunden ist, deren Enantiomere, Diastereoisomere sowie deren Additionssalze mit einer pharmazeutisch annehmbaren Säure oder Base.

**4.** Verbindungen der Formel (I) nach Anspruch 1, worin n den Wert 0 besitzt, deren Enantiomere, Diastereoisomere sowie deren Additionssalze mit einer pharmazeutisch annehmbaren Säure oder Base.

**5.** Verbindungen der Formel (I) nach Anspruch 1, worin X eine Gruppe C-$R_2$ darstellt, worin $R_2$ ein Wasserstoffatom oder eine geradkettige oder verzweigte ($C_1$-$C_6$)-Alkylgruppe darstellt, deren Enantiomere, Diastereoisomere sowie deren Additionssalze mit einer pharmazeutisch annehmbaren Säure oder Base.

**6.** Verbindungen der Formel (I) nach Anspruch 1, worin X ein Stickstoffatom bedeutet, deren Enantiomere, Diastereoisomere sowie deren Additionssalze mit einer pharmazeutisch annehmbaren Säure oder Base.

**7.** Verbindungen der Formel (I) nach Anspruch 1, worin A eine σ-Bindung oder eine geradkettige oder verzweigte ($C_1$-$C_6$)-Alkylengruppe und $G_1$ eine Pyrrolidinyl- oder Piperidinylgruppe bedeuten, wobei jede dieser Gruppen gegebenenfalls an einem ihrer Ringatome durch eine geradkettige oder verzweigte ($C_1$-$C_6$)-Alkylgruppe, ($C_3$-$C_7$)-Cycloalkylgruppe, geradkettige oder verzweigte ($C_3$-$C_7$)-Cycloalkyl-($C_1$-$C_6$)-alkylgruppe, Nitrilgruppe, Carboxygruppe, geradkettige oder verzweigte ($C_1$-$C_6$)-Alkoxycarbonylgruppe, Carbamoylgruppe (die gegebenenfalls durch eine oder zwei geradkettige oder verzweigte ($C_1$-$C_6$)-Alkylgruppen, ($C_3$-$C_7$)-Cycloalkylgruppen, gegebenenfalls substituierte Phenylgruppen und/oder gegebenenfalls substituierte Benzylgruppen substituiert ist), gegebenenfalls substituierte Arylgruppen, geradkettige oder verzweigte und gegebenenfalls substituierte Aryl-($C_1$-$C_6$)-alkylgruppen, gegebenenfalls substituierte Heteroarylgruppen oder geradkettige oder verzweigte, gegebenenfalls substituierte Heteroaryl-($C_1$-$C_6$)-alkylgruppen substituiert ist,
oder $G_1$ eine Gruppe

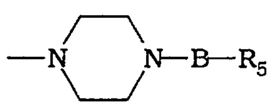 ,

worin B eine σ-Bindung oder eine geradkettige oder verzweigte ($C_1$-$C_6$)-Alkylengruppe und $R_5$ ein Wasserstoffatom oder eine geradkettige oder verzweigte ($C_1$-$C_6$)-Alkylgruppe, ($C_3$-$C_7$)-Cycloalkylgruppe, gegebenenfalls substituierte Arylgruppe, geradkettige oder verzweigte und gegebenenfalls substituierte Aryl-($C_1$-$C_6$)-alkylgruppe, gegebenenfalls substituierte Heteroarylgruppe oder geradkettige oder verzweigte und gegebenenfalls substituierte Heteroaryl-($C_1$-$C_6$)-alkylgruppe bedeuten, deren Enantiomere, Diastereoisomere sowie deren Additionssalze mit einer pharmazeutisch annehmbaren Säure oder Base.

**8.** Verbindung der Formel (I) nach Anspruch 1, worin A eine geradkettige oder verzweigte ($C_1$-$C_6$)-Alkylengruppe und $G_1$ eine Gruppe

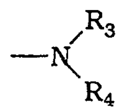

bedeuten, worin $R_3$ und $R_4$ unabhängig voneinander Wasserstoffatome, geradkettige oder verzweigte ($C_1$-$C_6$)-Alkylgruppen, ($C_3$-$C_7$)-Cycloalkylgruppen, geradkettige oder verzweigte ($C_3$-$C_7$)-Cycloalkyl-($C_1$-$C_6$)-alkylgruppen, geradkettige oder verzweigte und gegebenenfalls substituierte Aryl-($C_1$-$C_6$)-alkylgruppen und gegebenenfalls substituierte Arylgruppen bedeuten, deren Enantiomere, Diastereoisomere sowie deren Additionssalze mit einer pharmazeutisch annehbmaren Säure oder Base.

**9.** Verbindungen der Formel (I) nach Anspruch 1, worin die Triazolyl- oder die Triazolylmethylgruppe in der 6-Stellung des Indol- oder Indazolkerns gebunden ist, X eine Gruppe -CH- oder ein Stickstoffatom, $R_1$ ein Wasserstoffatom, A eine σ-Bindung oder eine geradkettige oder verzweigte ($C_1$-$C_6$)-Alkylengruppe und $G_1$ eine Pyrrolidinyl- oder Piperidinylgruppe, die gegebenenfalls an einem ihrer Ringatome durch eine geradkettige oder verzweigte ($C_1$-$C_6$)-Alkylgruppe, gegebenenfalls substituierte Arylgruppe, geradkettige oder verzweigte und gegebenenfalls substituierte Aryl-($C_1$-$C_6$)-alkylgruppe, gegebenenfalls substituierte Heteroarylgruppe oder geradkettige oder verzweigte und gegebenenfalls substituierte Heteroaryl-($C_1$-$C_6$)-alkylgruppe substituiert sind, oder $G_1$ eine 1-Piperazinylgruppe, die gegebenenfalls am Stickstoffatom in der 4-Stellung durch eine geradkettige oder verzweigte ($C_1$-$C_6$)-Alkylgruppe, gegebenenfalls substituierte Arylgruppe, geradkettige oder verzweigte und gegebenenfalls substituierte Aryl-($C_1$-$C_6$)-alkylgruppe, gegebenenfalls substituierte Heteroarylgruppe oder geradkettige oder verzweigte und gegebenenfalls substituierte Heteroaryl-($C_1$-$C_6$)-alkylgruppe substituiert ist, bedeuten,
oder A eine geradkettige oder verzweigte ($C_1$-$C_6$)-Alkylengruppe und $G_1$ eine Gruppe

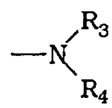

bedeuten, worin $R_3$ und $R_4$ unabhängig voneinander Wasserstoffatome oder geradkettige oder verzweigte ($C_1$-$C_6$)-Alkylgruppen bedeuten, deren Enantiomere, Diastereoisomere sowie deren Additionssalze mit einer pharmazeutisch annehmbaren Säure oder Base.

**10.** Verbindung der Formel (I) nach Anspruch 1, nämlich 1-(Dimethylaminoethyl)-6-([1,2,4]triazol-4-yl)-indol sowie dessen Additionssalze mit einer pharmazeutisch annehmbaren Säure.

**11.** Verbindung der Formel (I) nach Anspruch 1, nämlich 1-(Dimethylaminoethyl)-6-([1,2,4]triazol-4-yl)-indazol sowie dessen Additionssalze mit einer pharmazeutisch annehmbaren Säure.

**12.** Verbindung der Formel (I) nach Anspruch 1, nämlich 1-{3-[4-(5-Methoxypyrimidin-4-yl)-piperazin-1-yl]-propyl}-6-([1,2,4]triazol-4-yl)-indol sowie dessen Additionssalze mit einer pharmazeutisch annehmbaren Säure.

**13.** Verbindung der Formel (I) nach Anspruch 1, nämlich 1-{3-[4-(5-Methoxypyrimidin-4-yl)-piperazin-1-yl]-propyl}-6-([1,2,4]triazol-4-yl)-indazol sowie dessen Additionssalze mit einer pharmazeutisch annehmbaren Säure.

**14.** Verfahren zur Herstellung der Verbindungen der Formel (I), dadurch gekennzeichnet, daß man

- ♦ wenn in der herzustellenden Verbindung der Formel (I) X eine Gruppe C-$R_2$ darstellt, man als Ausgangsprodukt eine Verbindung der Formel (II/a) verwendet:

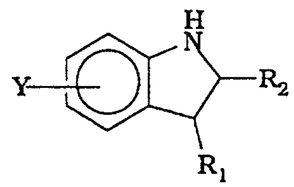 (II/a)

in der $R_1$ und $R_2$ die bezüglich der Formel (I) angegebenen Bedeutungen besitzen und Y eine Nitrogruppe darstellt, wenn in der herzustellenden Verbindung der Formel (I) n den Wert 0 besitzt, oder eine Hydroxymethylgruppe darstellt, wenn in der herzustellenden Verbindung der Formel (I) n den Wert 1 besitzt, welche man

- ***entweder*** mit einem Derivat der Formel (III/a):

$$Z - A - G_1 \qquad \text{(III/a)}$$

in der A und $G_1$ die bezüglich der Formel (I) angegebenen Bedeutungen besitzen und Z ein Halogenatom oder eine Formylgruppe darstellt, kondensiert zur Bildung einer Verbindung der Formel (IV):

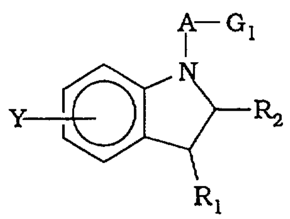 (IV)

in der A, Y, $G_1$, $R_1$, $R_2$ die oben angegebenen Bedeutungen besitzen, welche man einer Oxidationsreaktion unterwirft zur Bildung einer Verbindung der Formel (V/a):

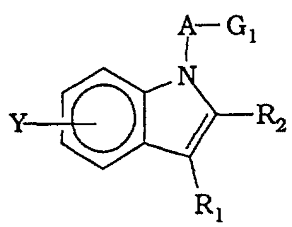 (V/a)

in der A, Y, $G_1$, $R_1$, $R_2$ die oben angegebenen Bedeutungen besitzen, deren Gruppe $G_1$, wenn sie eine Pyrrolidinyl-, Piperidyl- oder Piperazinylgruppe bedeutet, am Stickstoffatom in basischem Medium oder durch reduzierende Aminierung an einem Carbonylderivat substituiert werden kann,

- ***oder*** mit einem Derivat der Formel (III/b) in basischem Medium kondensiert:

$$Br - A - OH \qquad \text{(III/b)}$$

in der A die oben angegebenen Bedeutungen besitzt, zur Bildung einer Verbindung der Formel (IV'):

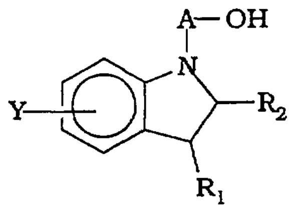

(IV')

in der Y, A, $R_1$, $R_2$ die oben angegebenen Bedeutungen besitzen, welche nach der Bromierung und Oxidation mit einem Derivat der Formel $G_1H$ kondensiert wird, worin $G_1$ die für die Formel (I) angegebenen Bedeutungen besitzt, zur Bildung einer Verbindung der Formel (V/a), wie sie oben definiert worden ist, oder

- ♦ wenn in der herzustellenden Verbindung der Formel (I) X ein Stickstoffatom bedeutet, man als Ausgangsmaterial eine Verbindung der Formel (II/b) verwendet:

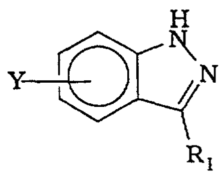

(II/b)

in der Y und $R_1$ die oben angegebenen Bedeutungen besitzen, welche

- ***entweder*** mit einem Derivat der Formel (III/c) in basischem Medium kondensiert wird:

$$\text{Hal - A - } G_1 \qquad \text{(III/c)}$$

in der A und $G_1$ die oben angegebenen Bedeutungen besitzen und Hal ein Halogenatom darstellt, zur Bildung einer Verbindung der Formel (V/b):

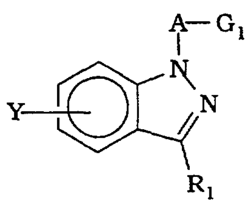

(V/b)

in der A, Y, $G_1$ und $R_1$ die oben angegebenen Bedeutungen besitzen, deren Gruppe $G_1$, wenn sie eine Pyrrolidinyl-, Piperidyl- oder Piperazinylgruppe bedeutet, am Stickstoffatom in basischem Medium oder durch eine reduzierende Aminierungsreaktion an einem Carbonylderivat substituiert werden kann,
- ***oder*** mit einem Derivat der Formel (III/b), wie sie oben definiert worden ist, kondensiert zur Bildung einer Verbindung der Formel (VI):

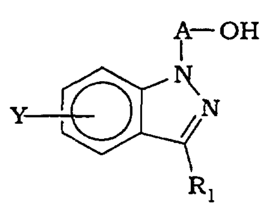

(VI)

in der Y, A und $R_1$ die oben angegebenen Bedeutungen besitzen, welche nach der Bromierung mit

einem Derivat der Formel $HG_1$ kondensiert wird, worin $G_1$ die bezüglich der Formel (I) angegebenen Bedeutungen besitzt, zur Bildung einer Verbindung der Formel (V/b), wie sie oben definiert worden ist, welche Verbindungen der Formeln (V/a) und (V/b) die Gesamtheit der Verbindungen der Formel (V) bilden:

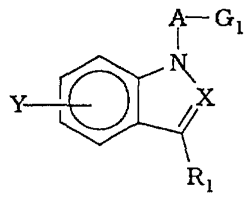

(V)

in der X, Y, A, $R_1$ und $G_1$ die bezüglich der Formel (I) angegebenen Bedeutungen besitzen und Y die oben angegebenen Bedeutungen aufweist,
welche durch Reduktion der Gruppe Y (wenn Y eine Hydroxymethylgruppe darstellt, über ein Zwischenprodukt vom Azido-Typ) zu einer Verbindung der Formel (VII) führt:

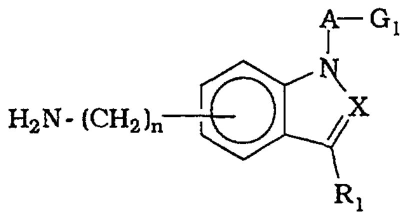

(VII)

in der n, X, A, $G_1$ und $R_1$ die oben angegebenen Bedeutungen besitzen, welche mit N,N-Dimethylformamid-azin in saurem Medium kondensiert wird zur Bildung der Verbindungen der Formel (I),
- welche man gegebenenfalls mit Hilfe einer klassischen Reinigungsmethode reinigt,
- welche man gegebenenfalls mit Hilfe einer klassischen Trennmethode in ihre Enantiomeren trennt,
- und die man gegebenenfalls in ihre Additionssalze mit einer pharmazeutisch annehmbaren Säure oder Base umwandelt,
- wobei es sich versteht, daß, wenn Y eine Hydroxygruppe enthält, diese letztere in Abhängigkeit von der Art der eingesetzten Reagenzien geschützt und wieder von der Schutzgruppe befreit werden kann.

**15.** Pharmazeutische Zubereitungen enthaltend als Wirkstoff mindestens eine Verbindung nach einem der Ansprüche 1 bis 13 allein oder in Kombination mit einem oder mehreren inerten, nichttoxischen, pharmazeutisch annehmbaren Trägermaterialien oder Bindemitteln.

**16.** Pharmazeutische Zubereitungen nach Anspruch 15 enthaltend mindestens einen Wirkstoff nach einem der Ansprüche 1 bis 13 zur Behandlung von Veneninsuffizienz und Erkrankungen, die verknüpft sind mit und/oder bei der Behandlung der Migräne und der Migräne, die mit Gefäßerkrankungen verbunden ist.